# EUROPEAN PATENT SPECIFICATION

(11) **EP 0 869 786 B1**
(45) Date of publication and mention of the grant of the patent: **05.03.2003**
(21) Application number: 96945283.8
(22) Date of filing: 20.12.1996
(51) Int. Cl.: C07D 273/08, C07D 413/06, A61K 31/395

(54) **PHARMACEUTICAL COMPOUNDS**
ARZNEISTOFFE
COMPOSES PHARMACEUTIQUES

(30) Priority: 22.12.1995 US 9145 P; 30.08.1996 US 25079 P
(43) Date of publication of application: 14.10.1998
(73) Proprietor: ELI LILLY AND COMPANY, Indianapolis, Indiana 46285 (US); UNIVERSITY OF HAWAII, Honolulu, HI 96822 (US); Wayne State University, Detroit, Michigan 48202 (US)
(72) Inventor: SHIH, Chuan, Carmel, IN 46033 (US)
(74) Representative: Burnside, Ivan John
(86) International application number: US9620633
(87) International publication number: WO97023211

(56) References cited:
- WO-A-95/17093
- WO-A-97/07798
- WO-A-97/08334
- WO-A-98/08506
- WO-A-98/08829
- US-A- 4 845 085
- US-A- 4 845 086
- US-A- 5 116 954
- US-A- 5 124 322

## Description

This invention relates to novel cryptophycin compounds useful as anti-microtubule agents.

Neoplastic diseases, characterized by the proliferation of cells not subject to the normal control of cell growth, are a major cause of death in humans and other mammals. Clinical experience in cancer chemotherapy has demonstrated that new and more effective drugs are desirable to treat these diseases. Such clinical experience has also demonstrated that drugs which disrupt the microtubule system of the cytoskeleton can be effective in inhibiting the proliferation of neoplastic cells.

The microtubule system of eucaryotic cells is a major component of the cytoskeleton and is a dynamic assembly and disassembly; this is heterodimers of tubulin are polymerized and form microtubule. Microtubules play a key role in the regulation of cell architecture, metabolism, and division. The dynamic state of microtubules is critical to their normal function. With respect to cell division, tubulin is polymerized into microtubles that form the mitotic spindle. The microtubules are then depolymerized when the mitotic spindle's use has been fulfilled. Accordingly, agents which disrupt the polymerization or depolymerization of microtubules, and thereby inhibit mitosis, comprise some of the most effective cancer chemotherapeutic agents in clinical use.

Further, such agents having the ability to disrupt the microtubule system can be useful for research purposes.

Such anti-mitotic agents or poisins may be classified into three groups on the basis of their molecular mechanism of action. The first group consists of agents, including colchicine and colcemid, which inhibit the formation of microtubules by sequestering tubulin. The second group consists of agents, including vinblastine and vincristine, which induce the formation of paracrystalline aggregates of tubulin. Vinblastine and vincristine are well known anticancer drugs: their action of disrupting mitotic spindle microtubules preferentially inhibits hyperproliferative cells. The third group consists of agents, including taxol, which promote the polymerization of tubulin and thus stabilizes microtubules.

The exhibition of drug resistance and multiple-drug resistance phenotype by many tumor cells and the clinically proven mode of action of anti-microtubule agents against neoplastic cells necessitates the development of anti-microtubule agents cytotoxic to non-drug resistant neoplastic cells as well as cytotoxic to neoplastic cells with a drug resistant phenotype. WO 95/17093 discloses certain cryptophycin compounds which inhibit the proliferation of hyperproliferative cells.

The present invention provides novel compounds having the desired anti-microtubule action. Such compounds can be prepared using total synthetic methods and are therefore well suited for development as pharmaceutically useful agents.

The presently claimed invention provides novel cryptophycin compounds of Formula I 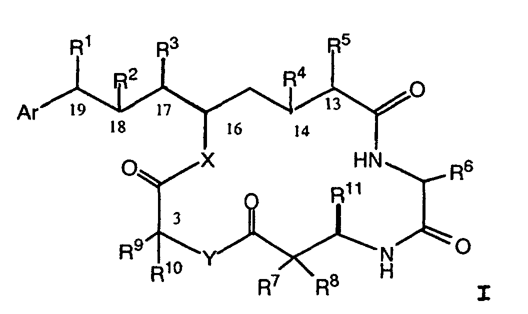 wherein
Ar is selected from the group consisting of phenyl, phenyl with a substituent selected from halogen, simple alkyl -OR^{12'} wherein R^{12'} is lower alkyl, and an aromatic group selected from furyl, pyrrolyl, thienyl, pyridyl, indolyl and naphthyl which is unsubstituted or substituted by a single group selected from halogen, lower alkyl and -OR^{12'} wherein R^{12'} is a simple alkyl group;
R¹ is selected from the group consisting of halogen, SH, amino, monoalkylamino, dialkylamino, trialkylammonium, alkylthio, dialkylsulfonium, sulfate, and phosphate;
R² is OH or SH; or
R¹ and R² may be taken together with C₁₈ and C₁₉ to form an epoxide ring, an aziridine ring, an episulfide ring, a sulfate ring, or monoalkylphosphate ring; or
R¹ and R² may be taken together to form a second bond between C₁₈ and C₁₉;
R³ is a lower alkyl group;
R⁴ is H or H₂;
R⁵ is H or H₂;
R⁴ and R⁵ may be taken together to form a second bond between C₁₃ and C₁₄;
R⁶ is selected from the group consisting of benzyl, hydroxybenzyl, alkoxybenzyl, halohydroxybenzyl, dihalohydroxybenzyl, haloalkoxybenzyl, and dihaloalkoxybenzyl group;
R⁷ is H or a lower alkyl group;
R⁸ is H or a lower alkyl group;
R⁷ and R⁸ may be taken together to form a spiro group;
R⁹ is H or a lower alkyl group;
R¹⁰ is H or a lower alkyl group;
R¹¹ is selected from the group consisting of simple alkyl, OH, phenyl, substituted phenyl, benzyl, and substituted benzyl;
X is O, NH or alkylamino;
Y is O, NH or alkylamino; or
a pharmaceutically acceptable salt or solvate thereof.

The present invention provides pharmaceutical formulations, a method for the preparation of a medicament for disrupting a microtubulin system using an effective amount of a compound of Formula **I**, a method for the preparation of a medicament for inhibiting the proliferation of mammalian cells comprising administering an effective amount of a compound of Formula **I**, and a method for the preparation of a medicament for treating neoplasia in a mammal comprising administering an effective amount of a compound of Formula I.

As used herein, the term "simple alkyl" shall refer to C₁-C₇ alkyl wherein the alkyl may be saturated, unsaturated, branched, or straight chain. Examples include, but are in no way limited to, methyl, ethyl, n-propyl, isopropyl, n-butyl, propenyl, sec-butyl, n-pentyl, isobutyl, tert-butyl, sec-butyl, methylated butyl groups, pentyl, tert pentyl, sec-pentyl and methylated pentyl groups.

The term "sulfate" shall have the meaning commonly attributed to the term by the artisan. Thus the term refers to a salt of sulfuric acid. For example, the term may refer to, but is not necessarily limited to, -SO₄ or -SO₃H.

The term "phosphate" shall have the meaning commonly attributed to the term by the artisan. Thus the term refers to a salt of phosphoric acid. For example, the term may refer to, but is not necessarily limited to, -PO₄ or -PO₄H₂.

As used herein, the term "substituted phenyl" shall refer to a phenyl group with from one to three non-hydrocarbon substituents which may be independently selected from the group consisting of simple alkyl, OR^{12'}, Cl, Br, F, and I, wherein R^{12'} is selected from simple alkyl. Most preferred, R^{12'} is C₁-C₃ alkyl. It is especially preferred that R^{12'} is CH₃.

As used herein, the term "substituted benzyl" shall refer to a benzyl group with from one to three non-hydrocarbon substitutents which may be independently selected from the group consisting of simple alkyl, Cl, Br, F, and I.

As used herein, "spiro group" refers to a cycloalkyl. For example, the spiro group is most preferredly cyclopropyl. The term "cycloalkyl" refers to a saturated C₃-C₈ cycloalkyl group.

As used herein "lower alkyl" means any alkyl group of one to five carbons and includes linear and non-linear hydrocarbon chains, including for example, but not limited to methyl, ethyl, propyl, isopropyl, butyl, isobutyl, tert-butyl, sec-butyl, methylated butyl groups, pentyl, tert pentyl, sec-pentyl, and methylated pentyl groups.

As used herein, "monoalkylamino" refers to a monoalkylamino group in which the alkyl portion of the group may be straight or branched. Examples of such groups include but are not limited to: methlamino, ehtylamino, 2-propylamino and t-butylamino.

As used herin, "dialkylamino" refers to a dialkylamino group in which the alkyl portion of the group may be straight or branched. Examples of such groups include, but are not limited to: dimethylamino, diethylamino, di(n-propyl)amino, di(iso-propyl)amino and methyl-n-propylamino. Likewise, "trialkylammonium" refers to a trialkylammonium group in which the alkyl portion of the group may be straight or branched.

As used herein, "dialkylsulfonium" refers to a dialkylsulfonium group in which the alkyl portion of the group may be straight or branched.

As used herein "epoxide ring" means a three-membered ring whose backbone consists of two carbons and an oxygen atom. As used herein, "aziridine ring" means a three-membered ring whose backbone consists of two carbon atoms and a nitrogen atom.

As used herein "episulfide ring" means a three-membered ring whose backbone consists of two carbon and a sulfur atom. As used herein "sulfate group" means a five membered ring consisting of a carbon-carbon-oxygen-sulfur-oxygen backbone with two additional oxygen atoms connected to the sulfur atom. As used herein, "monalkylphosphate ring" means a five membered ring consisting of a carbon-carbon-oxygen-phosphorous-oxygen backbone with two additional oxygen atoms, one of which bears a lower alkyl group, connected to the phosphorous atom.

As used herein, "halogen" refers to those members of the group on the periodic table historically known as halogens. Preferred halogens are Br, Cl, F, and I. Methods of halogenation include, but are not limited to, the addition of hydrogen halides, substitution at high temperature, photogenation, etc., and such methods are known to the skilled artisan.

As used herein, the term "mammal" shall refer to the Mammalia class of higher vertebrates. The term "mammal" includes, but is not limited to, a human. The term "treating" as used herein includes prophylaxis of the named condition or amelioration or elimination of the condition once it has been established.

Some preferred characteristics of this invention are set forth in the following tabular form wherein the features may be independently selected to provide preferred embodiments of this invention. The invention is in no way limited to the features described below:
A) R⁸ is ethyl, propyl, isopropyl, butyl, isobutyl or isopentyl;
B) R⁷ is ethyl, propyl, isopropyl, butyl, isobutyl, pentyl, or isopentyl;
C) R⁷ is H, R⁸ is methyl, R³ is methyl, and X and Y are not both O;
D) R³ is ethyl, propyl, isopropyl, butyl, isobutyl, pentyl or isopentyl;
E) R⁹ is methyl, ethyl, propyl, butyl, isobutyl, pentyl, or isopentyl;
F) R¹⁰ is methyl, ethyl, propyl, butyl, isobutyl, pentyl, or isopentyl;
G) a cryptophycin compound wherein at least one of the groups selected from the group consisting of C-3, C-6, C-7, C-10, C-16, C-17, C-18, C-19, and R¹¹ has R stereochemistry (numbering as set forth in claim 1 *infra.*) ;
H) a cryptophycin compound wherein at least one of the groups selected from the group consisting of C-3, C-6, C-7, C-10, C-16, C-17, C-18, C-19, and R¹¹ has S stereochemistry (numbering as set forth in claim 1 *infra*.);
I) Ar is phenyl with a substituent selected from the group consisting of hydrogen, halogen, simple alkyl, and -OR^{12'} wherein R^{12'} is lower alkyl;
J) a compound wherein the C-7 substituent is *R* configuration;
K) a compound wherein the C-7 substituent is *S* configuration;
L) R⁷, R⁸ are each hydrogen;
M) R⁷ and R⁸ are each selected from hydrogen or OH;
N) R¹¹ is simple alkyl;
O) R is selected from the group consisting of methyl, ethyl, n-propyl, and phenyl;
P) R¹ and R² form an epoxide ring;
Q) both X and Y are O;
R) R⁴ and R⁵ form a double bond;
S) R⁶ is substituted benzyl wherein one substituent is a halogen and one is an OR¹² group wherein R¹² is lower alkyl;
T) a compound of Formula I is used for disruption of a microtubulin system;
U) a compound of Formula I is used as an anti-neoplastic agent; and
V) a compound of Formula I is used for the treatment of cancer in a mammal.
W) Ar is para ethyl substituted phenyl; and
X) R⁷ and R⁸ join to form a spiro group.

The pharmaceutical or veterinary compositions disclosed herein are useful in a method of alleviating a pathological condition caused by hyperproliferating mammalian cells comprising administering to a subject an effective amount of said composition to inhibit proliferation of the cells. In a preferred embodiment the method further comprises administering to the subject at least one additional therapy directed to alleviating the pathological condition. In a preferred embodiment of the present invention, the pathological condition is characterized by the formation of neoplasms. In a further preferred embodiment of the present invention, the neoplasms are selected from the group consisting of mammary, small-cell lung, non-small-cell lung, colorectal, leukemia, melanoma, pancreatic adenocarcinoma, central nervous system (CNS), ovarian, prostate, sarcoma of soft tissue or bone, head and neck, gastric which includes pancreatic and esophageal, stomach, myeloma, bladder, renal, neuroendocrine which includes thyroid and non-Hodgkin's disease and Hodgkin's disease neoplasms.

As used herein "neoplastic" refers to a neoplasm, which is an abnormal growth, such growth occurring because of a proliferation of cells not subject to the usual limitations of growth. As used herein, "anti-neoplastic agent" is any compound, composition, admixture, co-mixture, or blend which inhibits, eleminates, retards, or reverses the neoplastic phenotype of a cell.

Chemotherapy, surgery, radiation therpy, therapy with biological response modifiers, and immunotherapy are currently used in the treatment of cancer. Each mode of therapy has specific indications which are known to those of ordinary skill in the art, and one or all may be employed in an attempt to achieve total destruction of neoplastic cells. Moreover, combination chemotherapy, chemotherapy utilizing compounds of Formula I in combination with other neoplastic agents, is also provided by the subject invention as combination therapy is generally more effective than the use of a single anti-neoplastic agent. Thus, a further aspect of the present invention provides compositions containing a therapeutically effective amount of at least one compound of Formula I, including the non-toxic addition salts thereof, which serve to provide the above recited benefits. Such compositions can also be provided together with physiologically tolerable liquid, gel, or solid carriers, diluents, adjuvants and excipients. Such carriers, adjuvants, and excipients may be found in the U.S. *Pharmacopeia*, Vol. **XXII** and National Formulary vol **XVII**, U.S. *Pharmacopeia* Convention. Inc. Rockville, MD (1989). Additional modes of treatment are provided in AHFS Drug Information, 1993 e. by the American Hospital Formulary Service, pp. 522-660. Each of these references are well known and readily available to the skilled artisan.

The present invention further provides that the pharmaceutical composition used to treat neoplastic disease contains at least one compound of Formula I and at least one additional anti-neoplastic agent. Anti-neoplastic agents which may be utilized in combination with Formula I compounds include those provided in the Merck Index **11**, pp 16-17, Merck & Co., Inc. (1989). The Merck Index is widely recognized and readily available to the skilled artisan.

In a further embodiment of this invention, antineoplastic agents may be antimetabolites which may include but are in no way limited to those selected from the group consisting of methotrexate, 5-fluorouracil, 6-mercaptopurine, cytosine, arabinoside, hydroxyurea, and 2-chlorodeoxyadenosine. In another embodiment of the present invention, the anti-neoplastic agents contemplated are alkylating agents which may include but are in no way limited to those selected from the group consisting of cyclophosphamide, mephalan, busulfan, paraplatin, chlorambucil, and nitrogen mustard. In a further embodiment, the anti-neoplastic agents are plant alkaloids which may include but are in no way limited to those selected from the group consisting of vincristine, vinblastine, taxol, and etoposide. In a further embodiment, the anti-neoplastic agents contemplated are antibiotics which may include, but are in no way limited to those selected from the group consisting of doxorubicin, daunorubicin, mitomycin C, and bleomycin. In a further embodiment, the anti-neoplastic agents contemplated are hormones which may include, but are in no way limited to those selected from the group consisting of calusterone, diomostavolone, propionate, epitiostanol, mepitiostane, testolactone, tamoxifen, polyestradiol phosphate, megesterol acetate, flutamide, nilutamide, and trilotane.

In a further embodiment, the anti-neoplastic agents contemplated include enzymes which may include, but are in no way limited to those selected from the group consisting of L-Asparginase and aminoacridine derivatives such as, but not limited to, amsacrine. Additional anti-neoplastic agents include those provided by Skeel, Roland T., "Antineoplastic Drugs and Biologic Response Modifier: Classification, Use and Toxicity of Clinically Useful Agents" Handbook of Cancer Chemotherapy (3rd ed.), Little Brown & Co. (1991).

These compounds and compositions can be administered to mammals for veterinary use. For example, domestic animals can be treated in much the same way as a human clinical patient. In general, the dosage required for therapeutic effect will vary according to the type of use, mode of administration, as well as the particularized requirements of the individual hosts. Typically, dosages will range from about 0.001 to 1000 mg/kg, and more usually 0.01 to 10 mg/kg of the host body weight. Alternatively, dosages within these ranges can be administered by constant infusion over an extended period of time, usually exceeding 24 hours, until the desired therapeutic benefits are obtained. Indeed, drug dosage, as well as route of administration, must be selected on the basis of relative effectiveness, relative toxicity, growth characteristics of tumor and effect of Formula I compound on cell cycle, drug pharmacokinetics, age, sex, physical condition of the patient and prior treatment.

The compound of Formula I, with or without additional anti-neoplastic agents, may be formulated into therapeutic compositions as natural or salt forms. Pharmaceutically acceptable non-toxic salts include base addition salts which may be derived from inorganic bases such as for example, sodium, potassium, ammonium, calcium, or ferric hydroxides, and such organic bases as isopropylamine, trimethylamine, 2-ethylamino ethanol, histidine and procaine. Such salts may also be formed as acid addition salts with any free cationic groups and will generally be formed with inorganic acids such as for example, hydrochloric or phosphoric acids or organic acids such as acetic, oxalic, tartaric and mandelic. Additional excipients which further the invention are provided to the skilled artisan for example in the U.S. *Pharmacopeia.*

The suitability of particular carriers for inclusion in a given therapeutic composition depends on the preferred route of administration. For example, anti-neoplastic compositions may be formulated for oral administration. Such compositions are typically prepared as liquid solution or suspensions or in solid forms. Oral formulation usually include such additives as binders, fillers, carriers, preservatives, stabilizing agents, emulsifiers, buffers, mannitol, lactose, starch, magnesium stearate, sodium saccharin, cellulose and magnesium carbonate. These compositions may take the form of solutions, suspensions, tablets, pills, capsules, sustained relsease formulations, or powders, and typically contain 1% to 95% of active ingedient. More preferably, the composition contains from about 2% to about 70% active ingredient.

Compositions of the present invention may be prepared as injectables, either as liquid solutions, suspensions, or emulsions; solid forms suitable for solution in or suspension in liquid prior to injection. Such injectables may be administered subcutaneously, intravenously, intraperitoneally, intramuscularly, intrathecally, or intrapleurally. The active ingredient or ingredients are often mixed with diluents, carriers, or excipients which are physiologically tolerable and compatible with the active ingredient(s). Suitable diluents and excipients are for example, water, saline, dextrose, glycerol, and combinations thereof. In addition, if desired, the compositions may contain minor amounts of auxilary substances such as wetting or emulsifying agents, stabilizing or pH buffering agents.

The compounds of Formula I are useful in methods for inhibiting the proliferation of mammalian cells by contacting these cells with a Formula I compound in an amount sufficient to inhibit the proliferation of the mammalian cell. A preferred embodiment is a method to inhibit the proliferation of hyperproliferative mammalian cells. For purposes of this invention "hyperproliferative mammalian cells" are mammalian cells which are not subject to the characteristic limitations of growth (programmed cell death for example). A further preferred embodiment is when the mammalian cell is human. The method further provides contacting the mammalian cell with at least one Formula I compound and at least one anti-neoplastic agent. The types of anti-neoplastic agents contemplated are discussed *supra.*

The compounds of Formula I are further useful in methods for inhibiting the proliferation of hyperproliferative cells with drug-resistant phenotypes, including those with multiple drug-resistant phenotypes, by contacting said cell with a compound of Formula I in an amount sufficient to inhibit the proliferation of a hyperproliferative mammalian cell. A preferred embodiment is when the mammalian cell is human. The method further provides contacting a Formula I compound and at least one additional anti-neoplastic agent, discussed *supra.*

The compounds of Formula I are also useful in a method for alleviating pathological conditions caused by hyperproliferating mammalian cells for example, neoplasia, by administering to a subject an effective amount of a pharmaceutical composition containing Formula I compound to inhibit the proliferation of the hyperproliferating cells. As used herein "pathological condition" refers to any pathology arising from the proliferation of mammalian cells that are not subject to the normal limitations of growth. Such proliferation of cells may be due to neoplasms as discussed *supra.*

In a further preferred embodiment the neoplastic cells are human. The present method provides methods of alleviating such pathological conditions utilizing a compound of Formula I in combination with other therapies, as well as other anti-neoplastic agents.

The effectiveness of the claimed compounds can be assessed using standard methods known to the skilled artisan. One such study provided the following results:

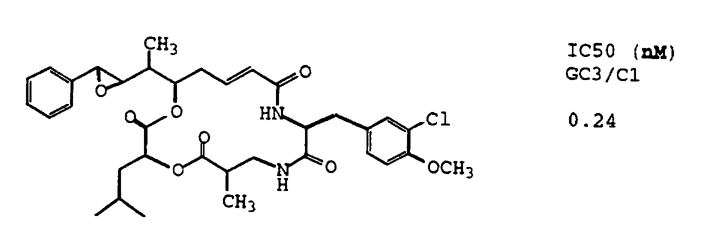

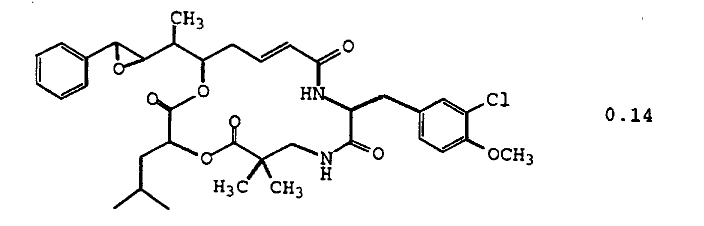

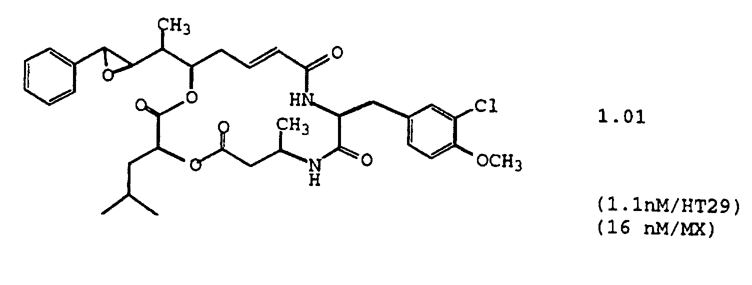

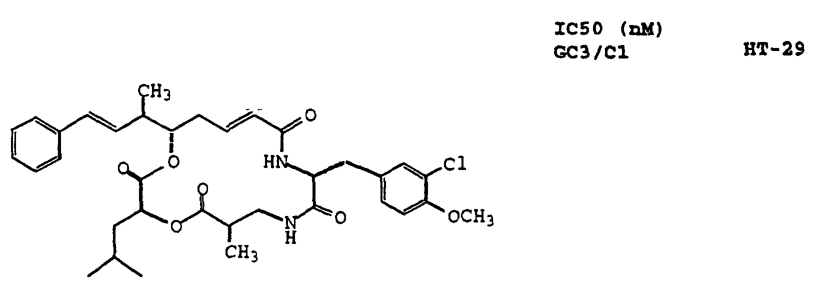

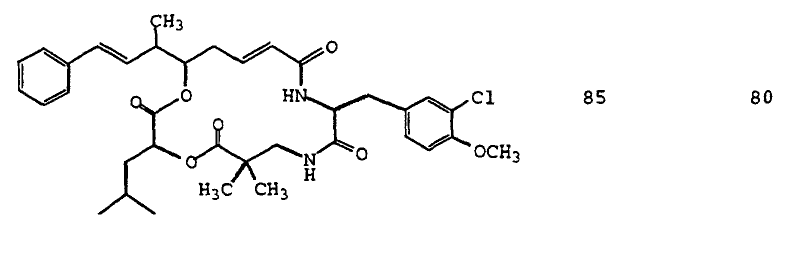

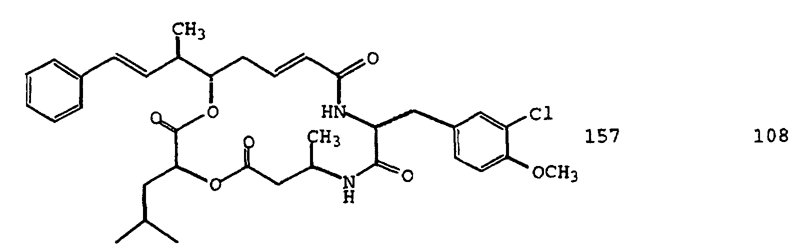

The compounds are screened for minimum inhibitory concentrations against KB, a human nasopharyngeal carcinoma cell line, LoVo, a human colorectal adenocarcinoma cell line. The Corbett assay, see Corbett, T.H. et al. Cytotoxic Anticancer Drugs: Models and Concepts for Drug Discovery and Development, pp 35-87, Kluwer Academic Publishers: Norwell, 1992. see also, Valeriote, et al. Discovery and Development of Anticancer Agents; Kluwer Academic Publishers, Norwell, 1993.

The most active compounds are further evaluated for cytotoxicity against four different cell types, for example a murine leukemia, a murine solid tumor, a human solid tumor, and a low malignancy fibroblast using the Corbett assay.

The compounds are further evaluated against a broad spectrum of murine and human tumors implanted in mice, including drug resistant tumors.

Tumor burden (T/C) (mean tumor burden in treated animals verses mena tumor burden in untreated animals) are used as a further assessment. T/C values that are less than 42% are considered to be active by National Cancer Institute Standards; T/C values less than 10% are considered to have excellent activity and potential clinical activity by National Cancer Institute standards.

Further, the compounds will be useful for disrupting the microtubule system.

The compounds of this invention can be prepared as illustrated using the following schemes. 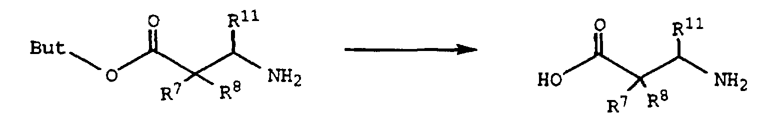 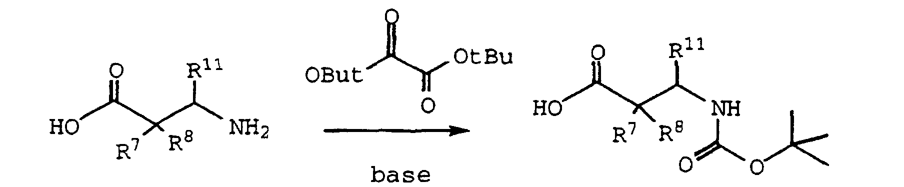 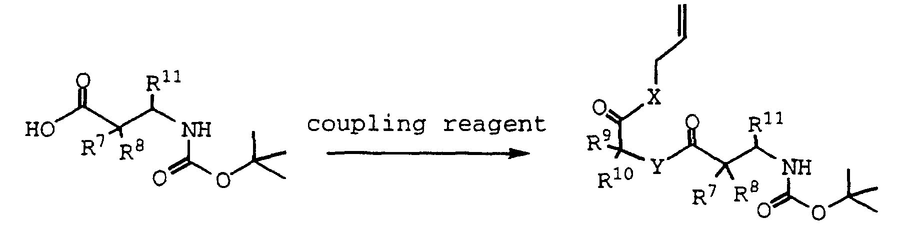 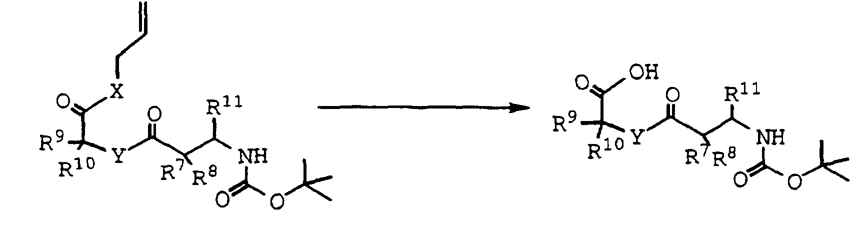 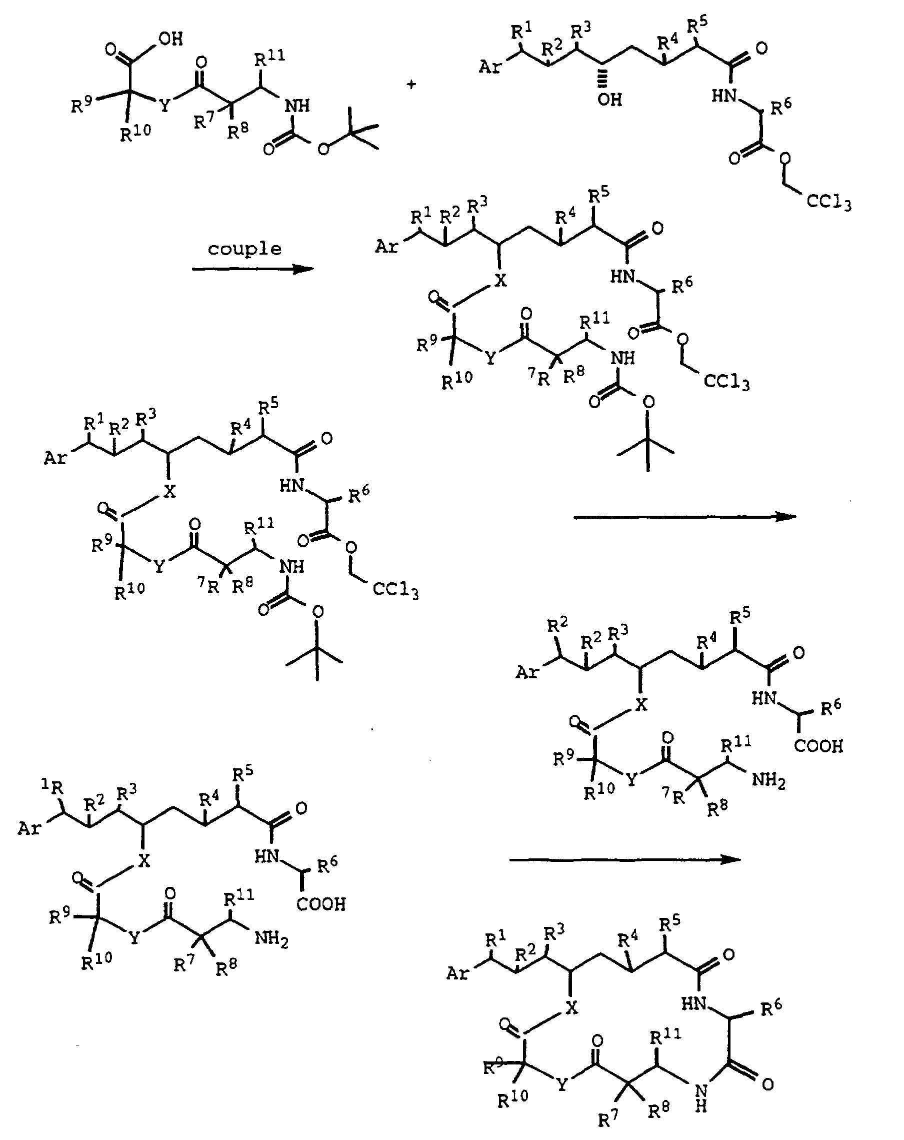 More specifically, compounds of this invention can be prepared as illustrated in the following schemes:

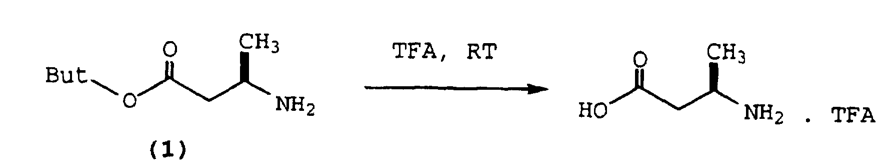

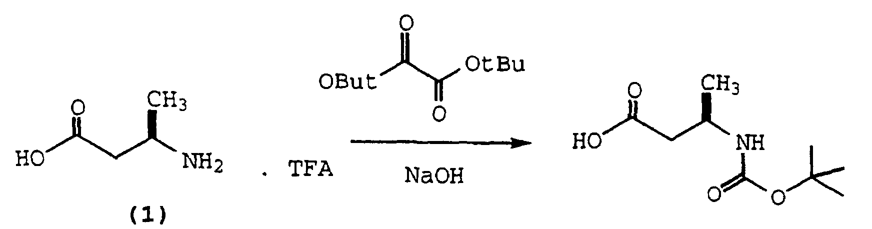

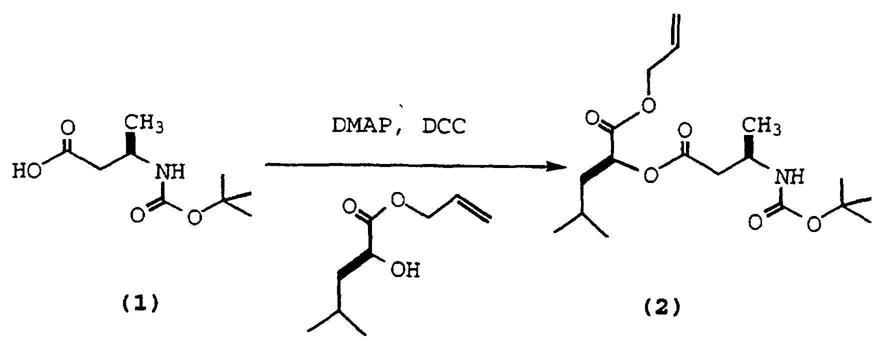

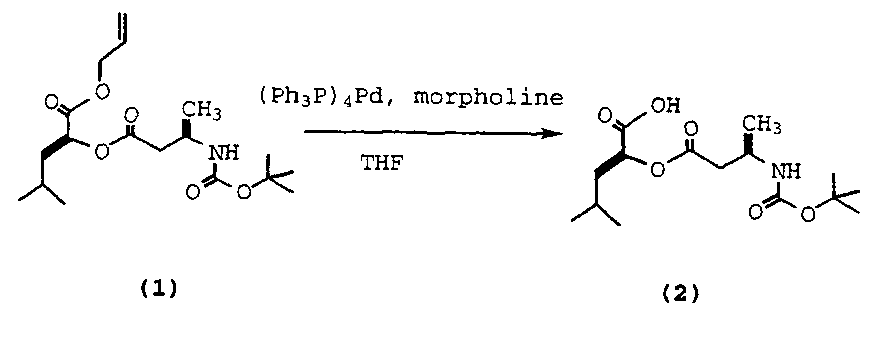

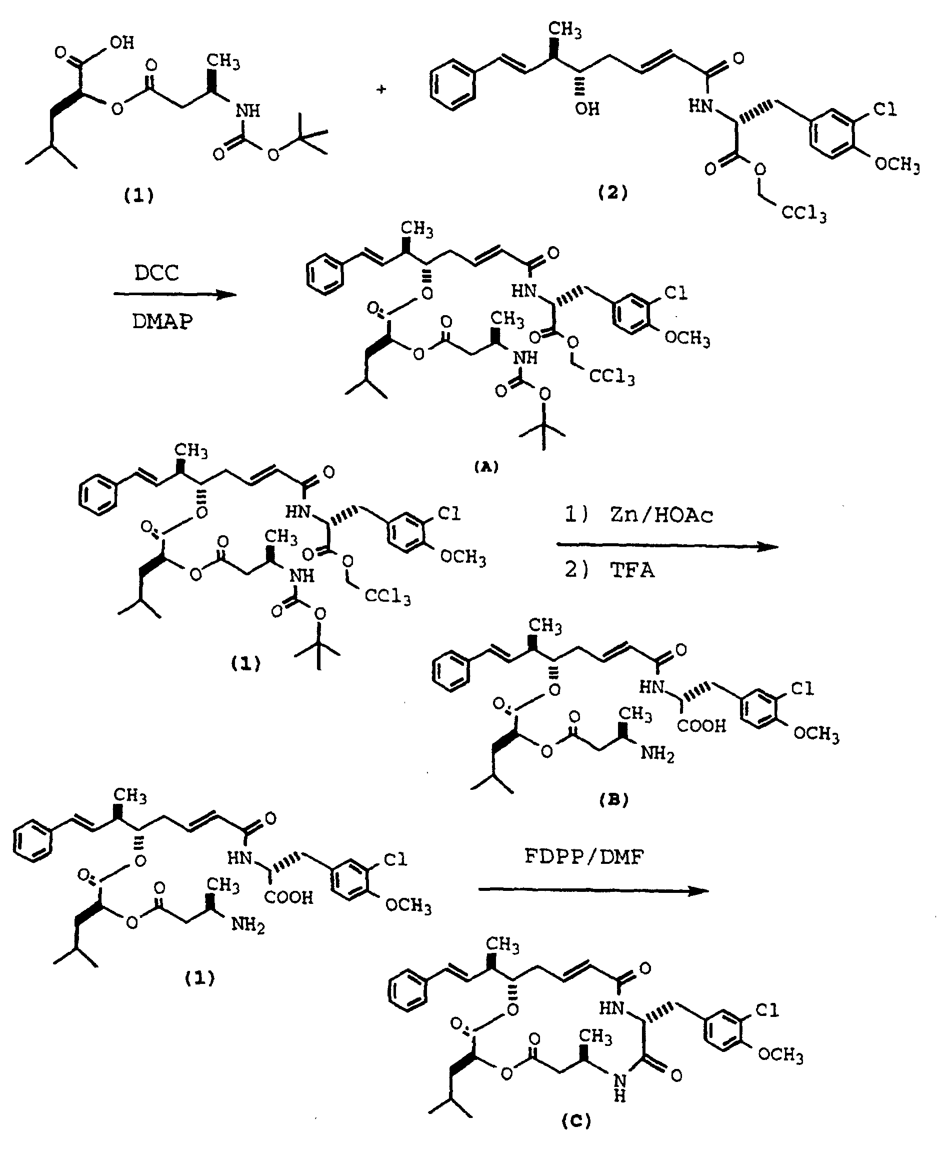

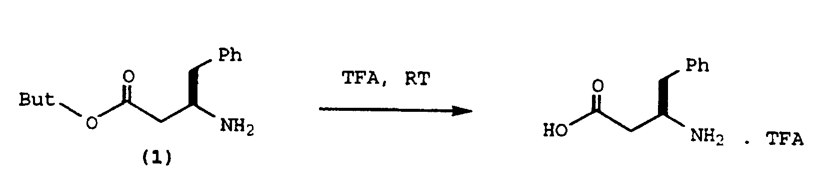

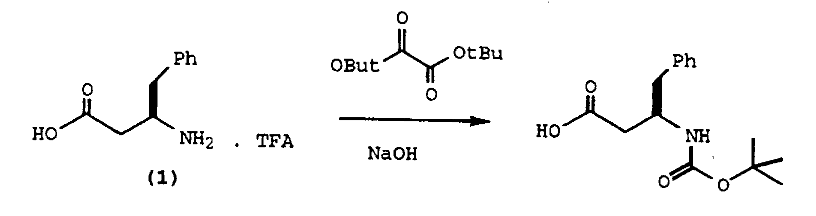

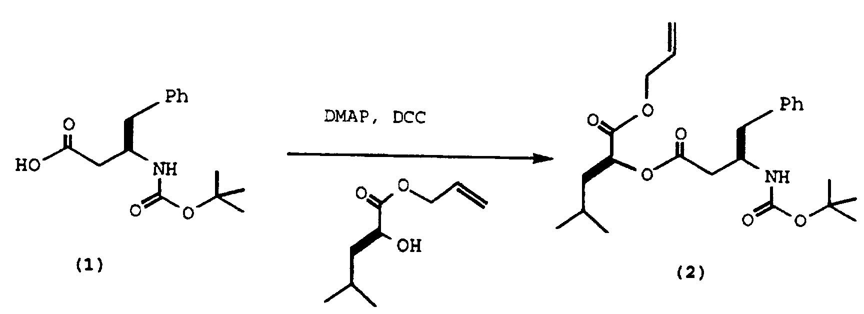

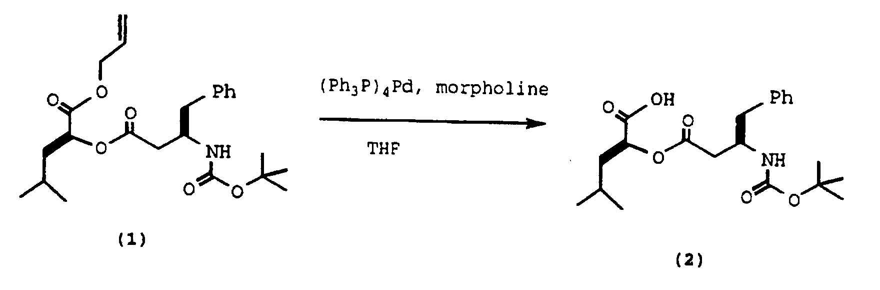

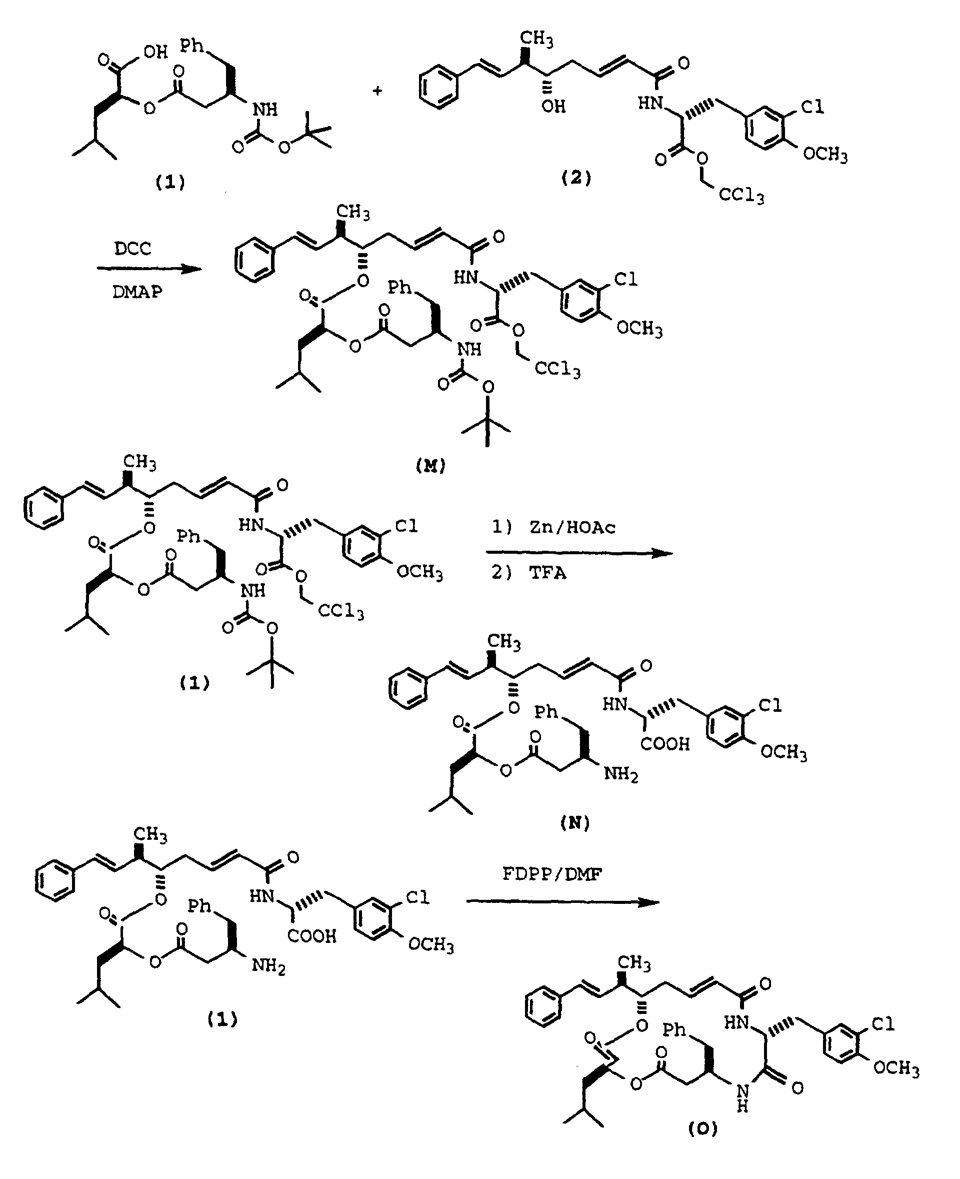

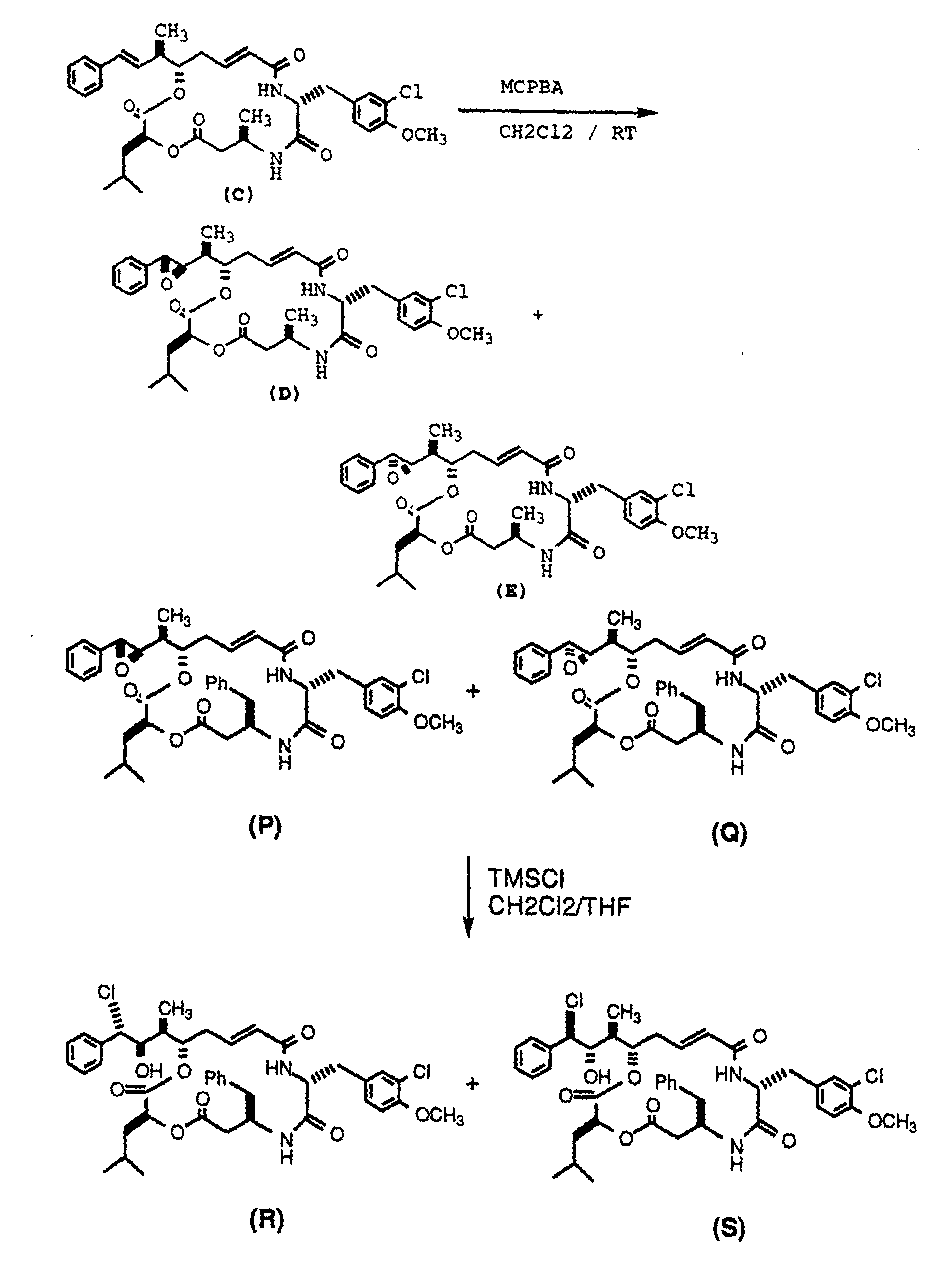

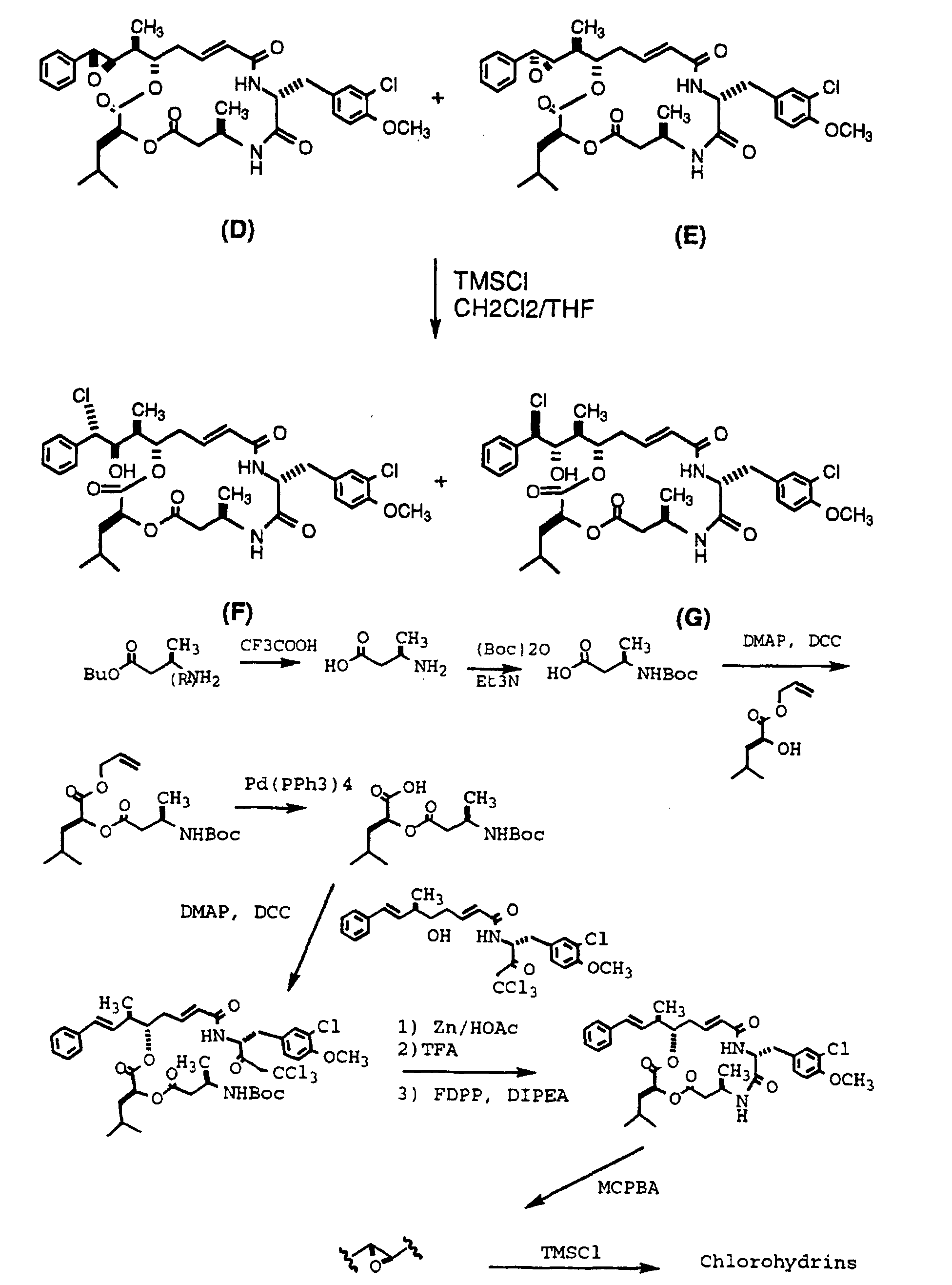

The artisan can utilize appropriate starting materials and reagents to prepare desired compounds using the guidance of the previous schemes and following examples.

To further illustrate the invention the following examples are provided. The scope of the invention is in no way to be construed as limited to or by the following examples.

### Preparation 1

### Synthesis of (3R)-benzyl-3-aminopropanoic acid (TFA salt)

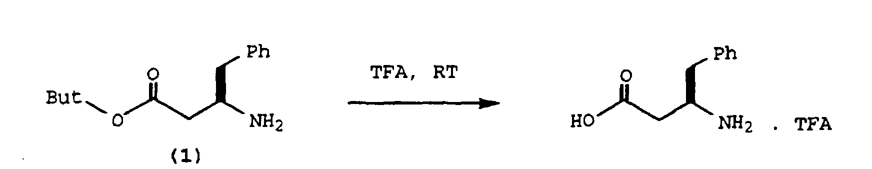

A sample of t-Butyl-3-(R)-benzyl-3-amino-propanoic acid **(1)** (purchased from oxford Asymmetry, England, >99% e.e) was dissolved in trifluoroacetic acid (herein "TFA") and then let stirred at room temperature for about 4 hours. The trifluoroacetic acid was removed in vacuo to give an oily residue which was then triturated with methanol to give a white solid.
TLC: Rf= (CHCl₃/CH₃OH/NH₄OH: 6:3.2:0.8)
IR (cm⁻¹) :
¹HNMR(300 MHz,DMSO-d6) d: 7.93 (bs, 2H), 7.32 (m, 5H). 3.63 (t, J= 7.2 Hz, 1H), 2.91 (dd, J= 5.9 Hz, J= 13.6 Hz, 2H), 2.77 (dd, J= 8.1 Hz, J= 13.6 Hz, 2H)
Anal: Calcd for C₁₂H₁₄NO₄: C, 49.15; H,4.81; N,4.78. Found: C, 48.87; H,4.73, N, 4.70.

### Preparation 2

### Synthesis of (3R)-benzyl-3-(tert-butoxycarbonyl)amino-propanoic acid

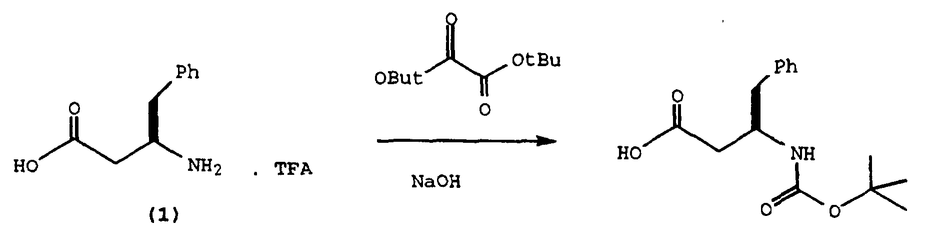

A sample of **(1)** was dissolved in 1,4-dioxane/H₂O/2.0 NaOH (2:2:1) at about 0°C (ice bath). To this was then added di-t-butyl-dicarboxylate and the ice bath was removed and the resulting reaction mixture was stirred at room temperature for about 18 hours. The reaction mixture was then concentrated to ca 10 ml and 25 ml of EtOAc was added. To this was then added 0.5 N NaHSO₄ to lower the pH of aqueous phase to ca. 2-3. The organic layer was then separated and the aqueous layer was extracted with EtOAc (20 ml x3). The combined EtOAc layer were then washed with water and brine and dried over NaSO₄. The solvent was then removed in vacuo to give a pale yellow solid.
TLC: Rf= (CHC₁₃/CH₃OH/NH₄OH: 6:3.2:0.8)
IR (cm⁻¹): 3361, 2985, 1670, 1686, 1526, 1266, 1168, 700.
UV (CH₃OH): 258 nm (e= 158).
OR: [a]_{D}= -136.71 (CHCl₃, C= )
¹HNMR(300 MHz.DMSO-d6) d: 7.20 (m, 5H), 6.75 (d, J= 8.6 Hz, 1H), 3.88 (m, 1H), 2.64 (d, J= 7.0 Hz, 2H), 2.28 (t, J= 5.1
Hz, 2H)1.27 (s, 9H).
Mass(FAB): 280 (M⁺+H).
Anal: Calcd for C₁₅H₂₁NO₄: C, 64.50; H,7.58; N,5.01. Found: C, 63.25; H, 7.35, N, 4.99.

### Preparation 3

### Synthesis of Allyl (2S)-2-[3'(tert-Butoxycarbonyl)amino-2'-(R)-benzylpropanoyloxy]-4-methylpentanoate: (2)

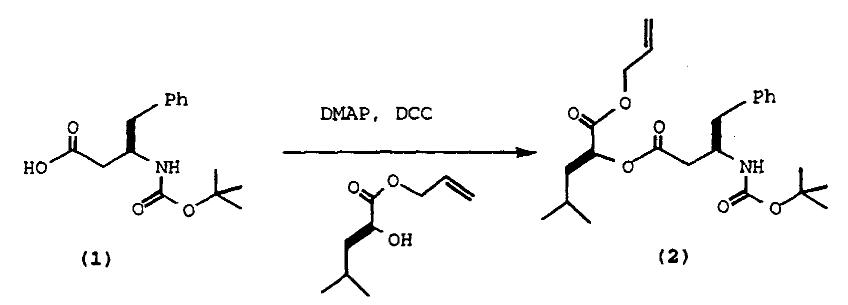

To a solution of allyl (2S)-2-hydroxy-4-methylpentanoate **(1)** and (1.2 eq) of (3R)-methyl-3-(tert-butoxycarbonyl)aminopropanoic acid in 10 ml of dry methylene chloride at 0°C (ice bath), was added (4.4mmol) of dicyclohexylcarbodiimide and then followed by (0.2 eq) of DMAP. The reaction mixture was then let stirred at room temperature for about 3 hours (TLC indicated the completion of the reaction). The reaction mixture was then filtered through a small pad of celite and the filtrate was washed with 5 % NaHCO₃, brine and dried over Na₂SO₄. The solvent was removed in vacuo and the residue was flash chromatographed on SiO₂ (15 % EtOAc/hexane) to give the product (%) of **(2)** as a clear oil.
TLC: Rf= (20 % EtOAc/hexane)
IR (cm⁻¹): 2961, 2933, 1742, 1715, 1497, 1366, 1249, 1170, 1127.
UV (CH3OH): 258 nm (e= 218).
OR: [a]_{D}= +7.55 (CHCl3, c= )
¹HNMR(300 MHz, CDCl₃) d: 7.25 (m, 5H), 5.89 (m, 1H), 5.20-5.36 (m, 3H), 5.10 (dd, J= 3.9 Hz, J= 9.6 Hz, 1H), 4.65 (d, J= 5.4 Hz, 2H), 4.15 (bs, 1H), 2.87 (m, 2H), 2.62 (dd, J= 5.6 Hz, J= 15.4 Hz, 1H), 2.50 (dd, J= 5.0 Hz, J= 15.4 Hz, 1H), 1.60-1.85 (m, 3H), 1.40 (s, 9H), 0.95 (d, J= 4.3 Hz, 3H), 0.93 (d, J= 4.3 Hz, 3H).
Mass(FAB): 434.4 (M⁺+H).
Anal: Calcd for C₂₄H₃₅NO₆: C, 66.49; H,8.14; N,3.23. Found: C, 66.32; H, 8.29, N, 3.42.

### Preparation 4

### Synthesis of (2S)-2-[3'(tert-Butoxycarbonyl)amino-2'-(R)-benzylpropanoyloxy]-4-methylpentanoic acid (2)

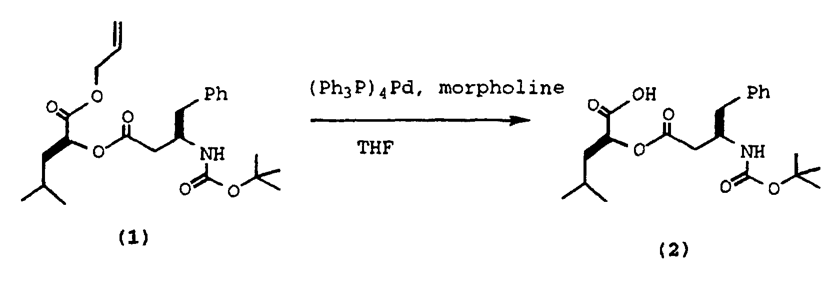

To a sample of 0.98 g (2.26 mmol) of **(1)**, 0.277 g (0.23mmol) of tetrakistriphenyl-palladium in 50 ml of dry THF was added 2.2 ml of anhydrous morpholine. The reaction mixture was then let stirred at room temperature for 1.5 h and TLC showed the disappearance of the starting material. The reaction mixture was then diluted with 30 ml of diethyl ether and washed with 50 ml of 1.0 N HCl (X2). The organic layer was then extracted with 2 X 50 ml of 5 % NaHCO₃. The combined aqueous layer was then acidified with 0.5 -1.0 N HCl to pH 3-4 and then extracted with ether (400 mL). The ether layer was then washed with brine and dried over Na₂SO₄ and concentrated in vacuo to give a pale yellow solid **(2)** weight 0.73 g (82 %).
TLC: Rf= (10 % CH3OH/CHCl3)
IR (cm⁻¹): 3034, 2962, 2934, 1727, 1709, 1498, 1455, 1393, 1369, 1253, 1198, 1165, 1127.
UV (CH3OH) 259 nm (e= 214).
¹HNMR(300 MHz, CDCl3) d: 7.20 (m 5H), 5.13 (m, 2H), 4.19 (bs, 1H), 2.84 (m, 2H), 2.40 -2.65 (m, 2H),1.60 -1.85 (m, 3H), 1.38 (s, 9H), 1.23 (d, J= 6.8 Hz, 3 H), 0.96 (d, J= 5.8 Hz, 3H), 0.93 (d, J= 5.8 Hz, 3H).
OR: [a]_{D} = +12.91 (CHCl3, c= )
Mass(FD): 394.4 (M⁺+H).
Anal: Calcd for C₂₁H₃₁NO₆: C, 64.10; H,7.94; N,3.56. Found: C, 64.16; H, 7.97, N, 3.43.

### preparation 5

### Synthesis of compound (M)

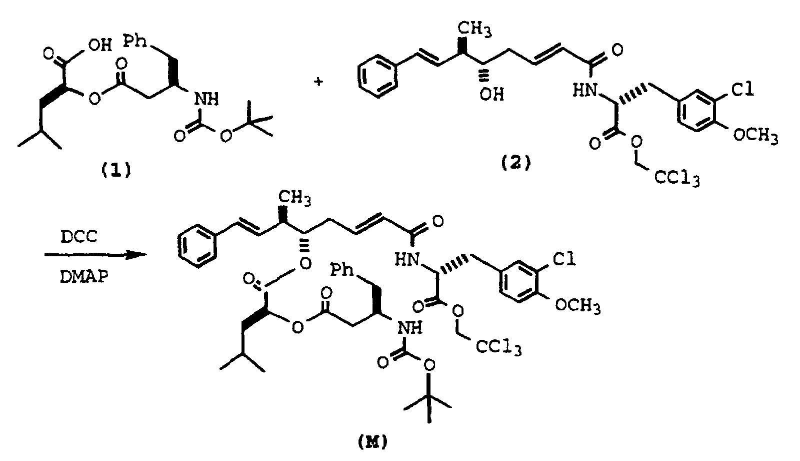

To a flame-dried 100 ml three-neck round bottom flask under argon was added 0.66 g (1.68 mmol, 1.5 eq) of (2S)-2-[3'(tert-Butoxycarbonyl)amino-2'-(R)-benzylpropanoyloxy]-4-methylpentanoic acid **(1)** and 0.66 g (1.13 mmol ) of compound **(2)** in 15 ml of dry methylene chloride at 0°C. To this solution was then added 34 mg of DMAP and 0.37 g (1.68 mmol, 1.50 eq) of dicyclohexylcarbodiimide and the resulting solution was stirred at Room Temperature for about 18 hours. TLC showed the completion of the reaction after 18hours and the white precipitate was filtered through a short pad of celite and the filtrate was diluted with 500 ml of ether. The organic layer was then washed with 50 ml of 1.0 N HCl followed by 50 ml of 5 % NaHCO₃. The solvent was then removed in vacuo to give a crude solid which weight ca. 1.22 g. This crude solid was then flash chromatographed on SiO₂ (35:65 EtOAc/hexane) to give a total of 0.958 g (88 %) of coupled product **(M)** as a foamed white solid.
TLC: Rf= (1:1 EtOAc/hexane)
IR (cm⁻¹): 1734, 1706, 1679, 1503, 1281, 1259, 1169.
UV (95 % EtOH): 230 nm (e= 21,823), 246 nm (e= 21,462).
¹HNMR(300 MHz,DMSO-d6) d: 7.16-7.34 (m, 11H), 7.04 (dd, J= 2.0 Hz, J= 8.4 Hz, 1H), 6.79 (d, J= 8.2 Hz, 1H), 6.74 (m, 1H), 6.55 (d, J= 8 Hz, 1H), 6.39 (d, J= 15.9hz, 1H), 5.96 (dd, J= 8.6 Hz, J= 15.8 Hz, 1H), 5.89 (d, J= 15.8 Hz, 1H), 5.04 (m, 3H), 4.93 (dd, J= 3.8 Hz, J= 8.0Hz, 1H), 4.76 (d, J= 11.9 Hz, 1H), 4.65 (d, J= 11.9 Hz, 1H), 4.12 (m, 1H), 3.82 (S, 3H), 3.15 (m, 1H), 3.08 (m, 1H), 2.85 (m, 2H),2.50 (m, 5H), 1.50-1.75 (m, 3H), 1.38 (s, 9H), 1.10 (d, J= 6.8 Hz, 3H), 0.85 (d, J= 6.4 Hz, 3H), 0.79 (d, J= 6.4 hz, 3H).
OR: [a]_{D}= +4.51 (CHCl₃, c= )
Mass(FAB): 965.4 (M⁺+ H).
Anal: Calcd for C₄₈H₅₈N₂O₁₀C₁₄: C, 59.76; H,6.06; N,2.90.
Found: C, 59.74; H, 6.19, N, 2.97.

### Preparation 6

### Synthesis of compound (N)

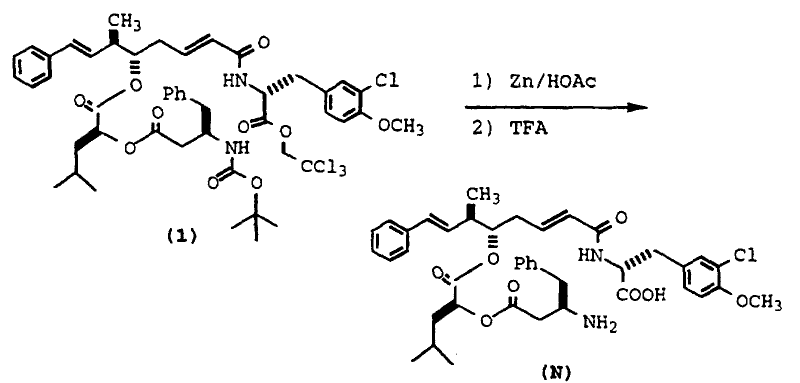

A sample of 0.80 g (0.83 mmol) of **(1)** was dissolved in 30 ml of glacial acetic acid and to this was added 3.0 g of zinc dust. The mixture was then sonicated (room temperature) for about 1.5 hour (TLC showed the complete consumption of the starting material). The zinc dust was then filtered away and the filtrate was concentrated to give a white solid. This crude solid was then dissolved immediately in 7.5 ml of trifluoroacetic acid and stirred at room temperature for about 2 hours. Trifluoroacetic acid (TFA) was then removed in vacuo and the oily solid was triturated with ether/hexane to give a white solid (0.814 g). This crude solid looks very good by TLC and 1HNMR, and this was then dissolved in ca. 50 ml of distilled water and triturated for 1 hour (with sonication). The white solid was then collected and dried in vacuo at about 50° C to give 0.55 g (78 % in two steps) of TFA salt of compound **(N)** (the aqueous filtrate from water trituration, however, did not contain any UV active material).
TLC: Rf= (10 % CH3OH/CHCL3)
IR (cm⁻¹): 2964, 1729, 1674, 1628, 1501, 1397, 1280, 1258, 1148, 1088.
UV (95 %EtOH): 232 nm (e= 23,051), 247 nm (e= 24,678).
¹HNMR(300 MHz,CD4OD) d: 7.14-7.37 (m, 12H), 7.06 (d, J= 1.9 Hz, J= 8.3 Hz, 1H), 6.86 (d, J= 8.4 Hz, 1H), 6.65 (m, 1H), 6.40 (d, J= 15.8 Hz, 1H), 5.92-6.05 (m, 2H), 4.97 (m, 2H), 4.51 (m, 1H), 3.73 (s, 3H), 3.66 (m, 2H), 2.80-3.15 (m, 4H), 2.40-2.72 (m, 3H), 1.40-1.70 (m, 3H), 1.09 (d, J= 6.7 Hz, 3H), 0.77 (d, J= 6.4 Hz, 3H), 0.68 (d, J= 6.4 hz, 3H).
OR: [a]_{D}= -29.80 (CH₃OH, c= )
Mass(FD): 733.4 (M⁺+H).
Anal: Calcd for C₄₁H₄₉N₂O₈Cl (TFA salt): C, 67.17; H,6.69;
N,3.82. Found: C, 68.04; H, 6.57, N, 3.47.

### Preparation 7

### Synthesis of (3R)-methyl-3-aminopropanoic acid (TFA salt)

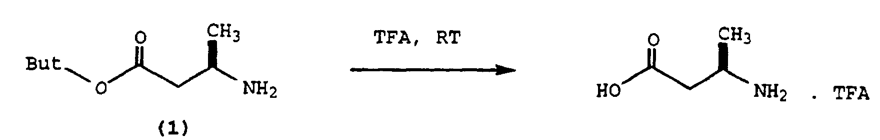

A sample of 750 mg (4.7mmol) of t-Butyl-3-(R)-methyl-3-amino-propanoic acid **(1)** (purchased from oxford Asymmetry, England, >99% e.e) was dissolved in 7.0 ml of trifluoroacetic acid (TFA) and then let stirred at room temperature for about 4 hours. The trifluoroacetic acid was removed in vacuo to give an oily residue which was then triturated with methanol to give a white solid, yield: 1.05 g (100%)
TLC: Rf=0.15 (CHCl₃/CH₃OH/NH₄OH: 6:3.2:0.8)
IR (cm⁻¹): 3286, 3092, 2996, 2914, 1714, 1654, 1504, 1448, 1237, 1196, 1143, 723.
¹HNMR(300 MHz,CD3OD) d: 3.61 (q, J= 6.6 Hz, 1H), 2.62 (t, J= 6.0 Hz, 2H), 1.32 (d, J=6.7 Hz, 3H)
Anal: Calcd for C₆H₁₀NO₄F₃: C, 33.19; H,4.64; N,6.45. Found: C, 33.32; H,4.64, N, 6.46.

### Preparation 8

### Synthesis of (3R)-methyl-3-(tert-butoxycarbonyl)amino-propanoic acid

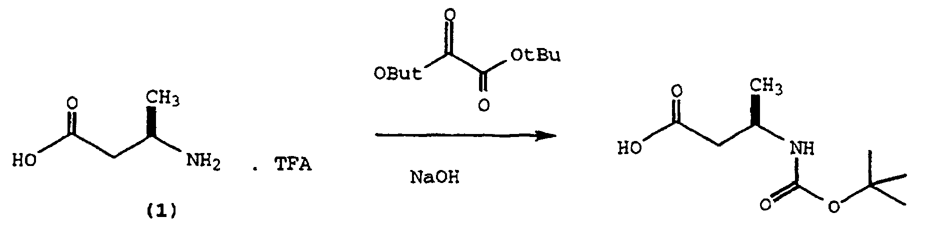

A sample of 1.0 g (4.6 mmol) of **(1)** was dissolved in 20 ml of 1,4-dioxane/H₂O/2.0 NNaOH (2:2:1) at 0°C (ice bath). To this was then added 1.16 ml (5.06 mmol) of di-t-butyldicarboxylate and the ice bath was removed and the resulting reaction mixture was let stirred at room temperature for 18h. The reaction mixture was then concentrated to ca 10 ml and 25 ml of EtOAc was added. To this was then added 0.5 N NaHSO₄ to lower the pH of aqueous phase to ca 2-3. The organic layer was then separated and the aqueous layer was extracted with EtOAc (20 ml x3). The combined EtOAc layer were then washed with water and brine and dried over NaSO₄. The solvent was then removed in vacuo to give a pale yellow solid. Yield: 0.88g (94%).
TLC: Rf=0.52 (CHCl₃/CH₃OH/NH₄OH: 6:3.2:0.8)
IR (cm⁻¹): 2981, 1711, 1504, 1368, 1244, 1166.
¹HNMR(300 MHz,DMSO-d6) d:3.74 (m, 1H), 2.35 (dd, J= 6.3 Hz, J=15.2 Hz, 1H), 2.16 (dd, J=7.6 Hz, J= 15.2Hz, 1H)1.33 (s, 9H), 0.99 (d, J= 6.6 Hz, 3H).
Mass(FAB): 204.2 (M⁺+H), 223.1 (M⁺+Na).
Anal: Calcd for C₉H₁₇NO₄: C, 53.19; H,8.43; N,6.89. Found: C, 53.42; H, 8.69, N, 6.77.

### Preparation 9

### Synthesis of Allyl (2S)-2-[3'(tert-Butoxycarbonyl)amino-2'-(R)-metbylpropanoyloxy]-4-methylpentanoate: (2)

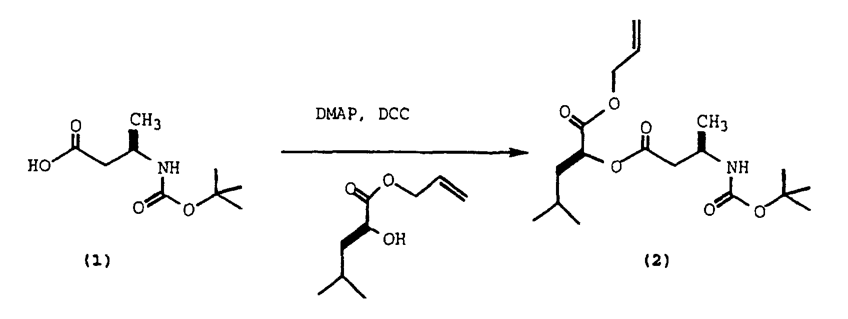

To a solution of 0.81 g (3.98 mmol) of allyl (2S)-2-hydroxy-4-methylpentanoate **(1)** and 0.82 g (4.78 mmol, 1.2 eq) of (3R)-methyl-3-(tert-butoxycarbonyl)amino-propanoic acid in 10 ml of dry methylene chloride at 0°C (ice bath), was added 0.91 g (4.4mmol) of dicyclohexylcarbodiimide and then followed by 0.13 g (0.96 mmol, 0.2 eq) of DMAP. The reaction mixture was then stirred at room temperature for about 3 hours (TLC indicated the completion of the reaction). The reaction mixture was then filtered and the filtrate was washed with 5 % NaHCO₃, brine and dried over Na₂SO₄. The solvent was removed in vacuo and the residue was flash chromatographed on SiO₂ (15 % EtOAc/hexane) to give 1.3 g (91%) of **(2)** as a clear oil.
TLC: Rf=0.48 (20 % EtOAc/hexane)
IR (cm⁻¹): 3442, 2963, 2937. 2874, 1738, 1706, 1503, 1469, 1456, 1391, 1368, 1341, 1274, 1239, 1169, 1121, 1104, 1013, 112, 930.
¹HNMR(300 MHz,DMSO-d6) d: 6,76 (d, J=7.7 Hz, 1H), 5.84 (m, 1H), 5.26 (d, J= 17.5Hz, 1H), 5.18 (d, J= 10.4 Hz, 1H), 4.89 (dd, J= 4.0 Hz, J= 9.0 Hz, 1H), 4.56 (d, J=4.9 Hz, 2H), 3.77 (m, 1H), 2.55 (dd, J= 6.2 Hz, J= 15 Hz, 1H), 2.31 (dd, J= 7.9 Hz, J= 15 Hz, 1H), 1.69 (m, 2H), 1.54 (m, 1H), 1.33 (s, 9H), 1.01 (d, J= 6.6Hz, 3H), 0.87 (d, J= 6.2 Hz, 3H), 0.84 (d, J= 6.3 Hz, 3H).
Mass(FAB): 358.2 (M⁺+H).
Anal: Calcd for C₁₈H₃₁NO₆: C, 60.48; H,8.74; N,3.92. Found: C, 60.50; H, 8.96, N, 3.66.

### Preparation 10

### Synthesis of (2S)-2-[3'(tert-Butoxycarbonyl)amino-2'-(R)-methylpropanoyloxy]-4-methylpentanoic acid (2):

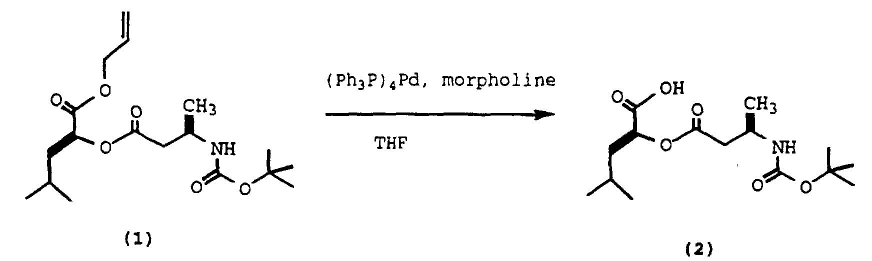

To a sample of 1.23 g (3.44 mmol) of **(1)**, 0.40 g (0.344mmol) of tetrakistriphenyl-palladium in 70 ml of dry THF was added 3.31 ml of anhydrous morpholine. The reaction mixture was then let stirred at room temperature for 1.5 h and TLC showed the disappearance of the starting material. The reaction mixture was then diluted with 30 ml of diethyl ether and washed with 200 ml of 1.0 N HCl. The organic layer was then extracted with 3 X 200 ml of 5 % NaHCO₃. The combined aqueous layer was then acidified with 0.5 -1.0 N HCl to pH 3-4 and then extracted with ether (400 mL). The ether layer was then washed with brine and dried over Na₂SO₄ and concentrated in vacuo to give a pale yellow solid **(2)** weight 1.02 g (93 %).
TLC: Rf=
IR (cm⁻¹): 2980, 2963, 2934, 2873, 1727, 1504, 1456, 1411, 1392, 1369, 1342, 1245, 1168, 1128, 1104, 1065.
¹HNMR(300 MHz,DMSO-d6) d: 5.09 (m, 1H), 5.01 (m, 1H), 4.08 (m, 1H), 2.61 (m, 2H), 1.74 (m, 3H), 1.44 (s, 9H), 1.23 (d, J= 6.8 Hz, 3 H), 0.96 (d, J= 6.2 Hz, 3H), 0.93 (d, J= 6.2 Hz, 3H).
OR: [a]_{D}= +18.045 (CHCl₃, c= )
Mass(FAB): 318.2 (M⁺+H), 340.2 (M⁺+ Na).
Anal: Calcd for C₁₅H₂₇NO₆: C, 56.77; H,8.57; N,4.41. Found: C, 57.82; H, 9.08, N, 4.11.

### Example 1

### Synthesis of compound (O)

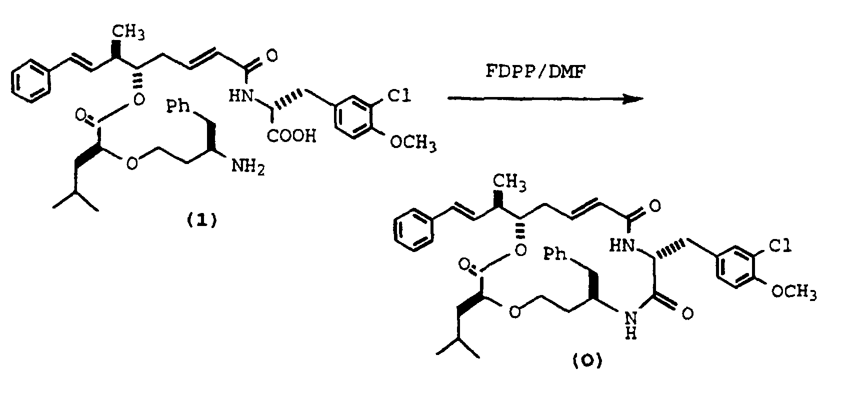

A sample of 0.295 mmol of **(1)** was dissolved in 50 ml of dry DMF under argon atmosphere and to this was then added 1.3 eq of pentafluorophenyldiphenylphosphinate (hereinafter "FDPP"). The resulting reaction mixture was then stirred at ambient conditions for about 18h. Another aliquot of FDPP was added after 18 h if there was unreacted starting material. The reaction was completed after stirring at room temperature for another 4 h. Dimethylformamide (hereinafter "DMF") was then removed in vacuo and the residue triturated with hexane to give a crude solid. This solid was then flash chromatographed on SiO₂ (5 % CH₃OH/CHCl₃) to give cyclized compound **(O)** as a solid.
The product was characterized by NMR, elemental analysis and mass spectrometry to confirm the product identity.

### Example 2

### Synthesis of epoxides (P) and (Q)

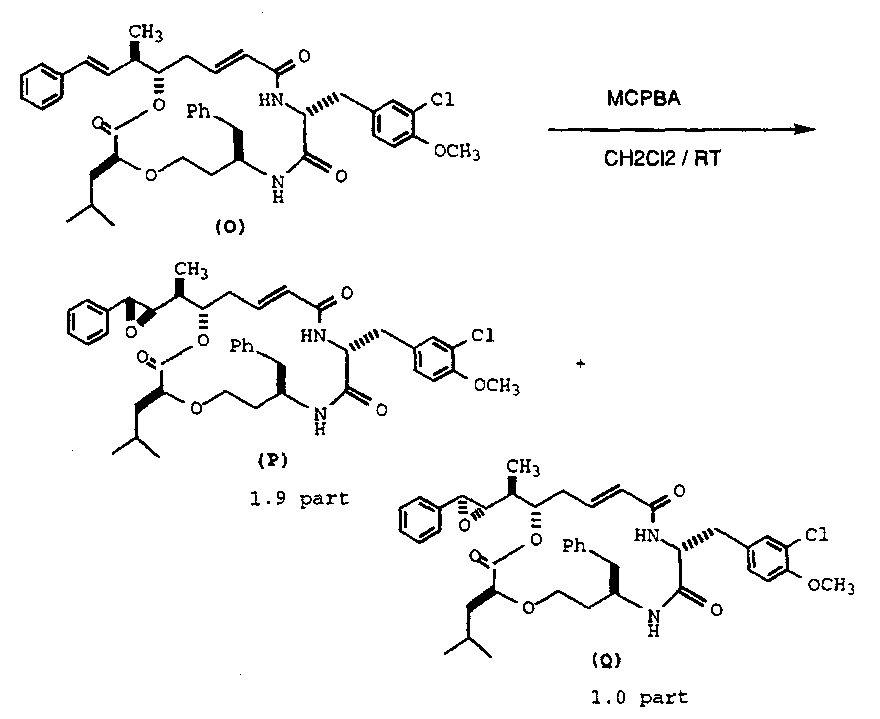

A (0.168 mmol) sample of compound **(O)** was dissolved in 6 ml of dry methylene chloride, to this was added 2.0 eq of *m*-chloroperoxybenzoic acid (hereinafter referred to as "MCPBA") at room temperature. This is stirred under argon for 18 about hours. The reaction mixture was checked by HPLC (2X4.6mmX15cm Novapak C-18 column, 75/25 CH3CN:H2O, 1.0 ml/min, monitored at 254 nm). An additional equivalent of MCPBA was added to utilize unreacted starting material and the reaction again monitored by HPLC. About 1.5 ml of the sample (ca 30 % eq) was removed and used immediately for the chlorohydrin reaction for the next step (see Example 3). Of the remaining 70 % reaction mixture, it was then diluted with methylene chloride, washed with 5%NaHCO3 (20mlX2) to remove the unreacted MCPBA and metachlorobenzoic acid. The organic layer was dried and concentrated in vacuo.

### Example 3

### Synthesis of chlorohydrin compounds (R) and (S)

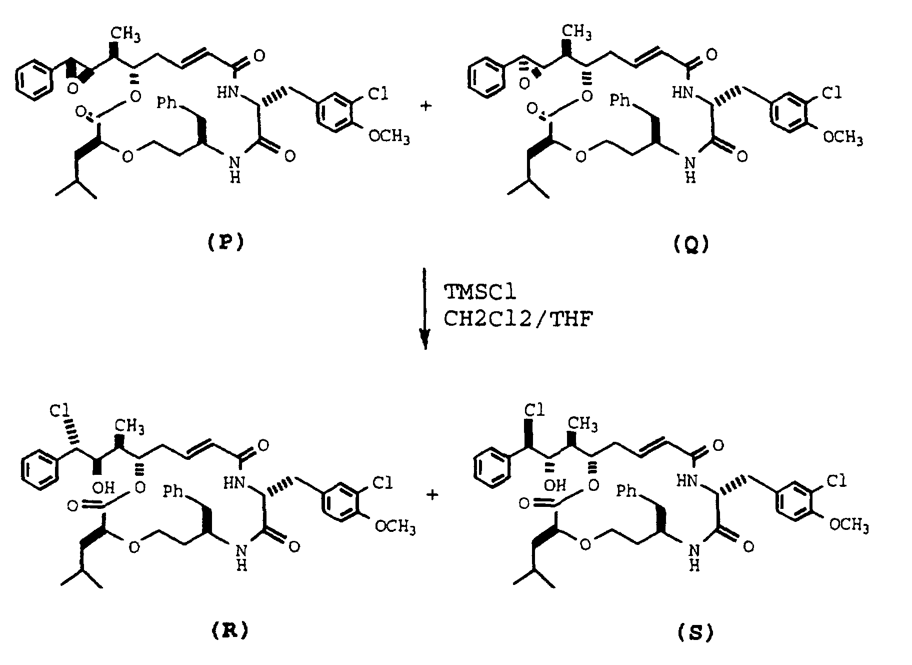

To the 1.5 ml methylene chloride solution obtained from the previous MCPBA reaction of Example 2 is added another 1.0 ml of dry THF. The reaction mixture is kept under argon atmosphere and cooled to -60 C. To this is added 5.0 eq of trimethylchlorosilane. The reaction is followed by TLC (5 % CH3OH/CHCl3). The reaction is stirred at -60 C for about 3 h before another 100ul of TMSCl is added. The reaction is then terminated after stirring at -40 C for about another 3 h. The solvent is then removed in vacuo to give a solid which is further purified by preparative reversed phase HPLC using conditions specified below:
solvent: CH3CN/H2O:
flow rate: 5.0 ml/min
UV: 254 nm

This gives the two chlorohydrins **(R)** and **(S)**:
Structures are verified using NMR, elemental analysis, and mass spectometry.

### Example 4

### Synthesis of compound (A)

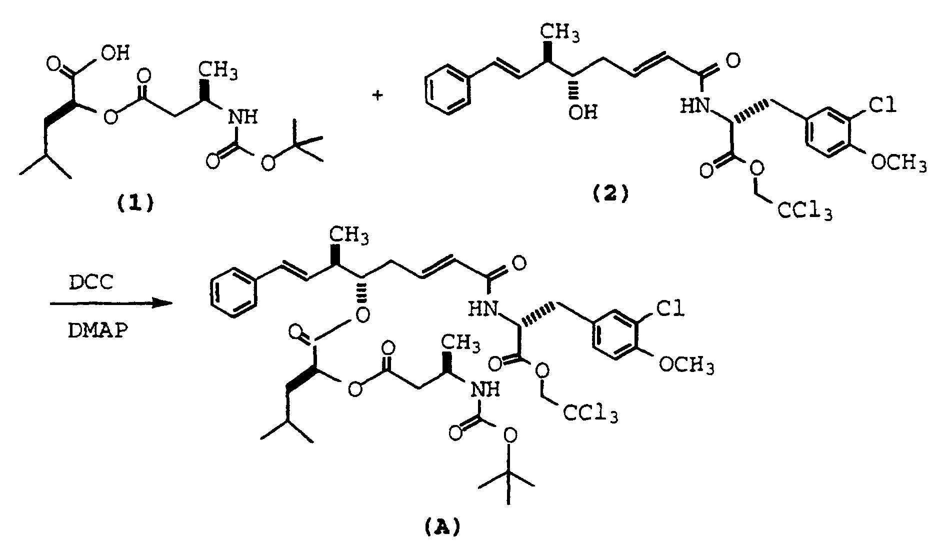

To a flame-dried 100 ml three-neck round bottom flask under argon was added 0.80 g (2.52 mmol, 1.5 eq) of (2S)-2-[3'(tert-Butoxycarbonyl)amino-2'-(R)-methylpropanoyloxy]-4-methylpentanoic acid **(1)** and 0.99 g (1.68 mmol ) of compound **(2)** in 50 ml of dry methylene chloride at 0°C. To this solution was then added 50 mg of DMAP and 0.52 g (2.52 mmol, 1.50 eq) of dicyclohexylcarbodiimide and the resulting solution was let stirred at RT for 4 h. TLC showed the completion of the reaction after 4h and the white precipitate was filtered through a short pad of celite and the filtrate was diluted with 500 ml of ether. The organic layer was then washed with 50 ml of 1.0 N HCl followed by 50 ml of 5 % NaHCO3. The solvent was then removed in vacuo to give a crude solid which weight ca. 1.70 g. This crude solid was then flash chromatographed on SiO2 (5 % EtOAc/methylene chloride) to give a total of 1.06 g (72 %) of coupled product **(A)** as a foamed solid.
TLC: Rf= 0.52 (1:1 EtOAc/hexane)
IR (cm⁻¹): 2964, 1743, 1713, 1677, 1642, 1504, 1367, 1281, 1259, 1170, 1127, 1066.
UV (CH3OH): 246 nm (e= 21,047).
¹HNMR(300 MHz,DMSO-d6) d: 7.31 (m, 5H), 7.19 (d, J= 2.0 Hz, 1H), 7.06 (dd, J= 2.0Hz, J= 8.4 Hz, 1H), 6.82 (d, J= 8.4 Hz, 1H), 6.65-6.78 (m, 2H), 6.38 (d, J= 15.9 Hz, 1H), 6.00 (dd, J= 8.7 Hz, J= 15.9 Hz, 1H), 5.89 (d, J= 15.6 Hz, 1H), 5.03 (m, 2H), 4.90 (dd, J= 3.7 Hz, J= 9.9Hz, 1H), 4.79 (d, J= 11.9 Hz, 1H), 4.69 (d, J= 11.9 Hz, 1H), 4.00 (m, 1H), 3.85 (S, 3H), 3.14 (m, 2H), 2.52 (m, 6H), 1.50-1.75 (m, 3H), 1.41 (s, 9H), 1.18 (d, J= 6.7 Hz, 3H), 1.10 (d, J= 6.8 Hz, 3H), 0.84 (d, J= 6.4 Hz, 3H), 0.79 (d, J= 6.4 hz, 3H).
OR: [a]_{D}= +0.792 (CHCl3, c= )
Mass(FD): 888.8 (M⁺).
Anal: Calcd for C42H54N2O10C14: C, 56.76; H,6.12; N,3.15. Found: C, 56.54; H, 6.16, N, 3.11.

### Example 5

### Synthesis of compound (B)

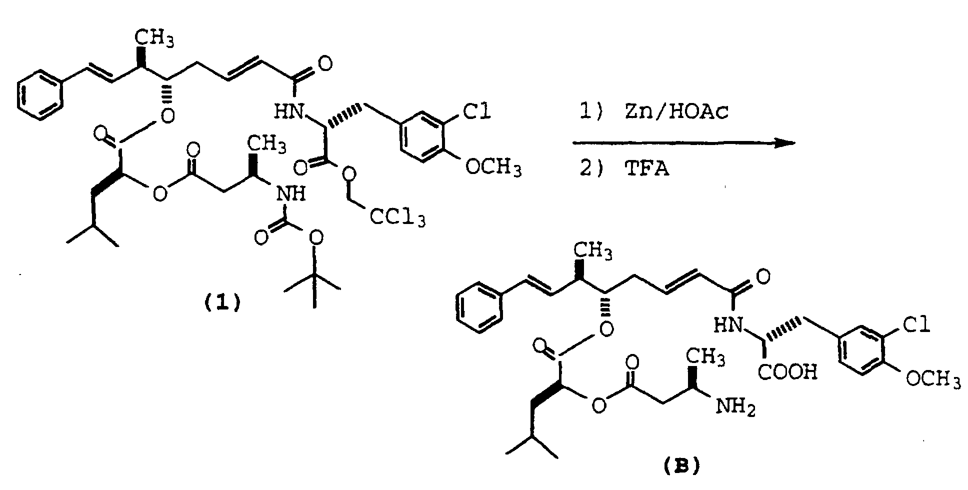

A sample of 0.98 g (1.12 mmol) of **(1)** in 40 ml of glacial acetic acid and to this was added 3.9 g of zinc dust. The mixture was then sonicated (room temperature) fro 45 min (TLC showed the completion of the starting material). The zinc dust was then filtered away through a short pad of celite and the filtrate was concentrated to give a white solid. This crude solid was then dissolved immediately in 50 ml of trifluoroacetic acid and let stirred at room temperature for 2 h. TFA was then removed in vacuo and the oily solid was triturated with ether/hexane to give a white solid (1.30 g). This crude solid ws then flash chromatographed on SiO2 (10 % CH3OH/CHCl3) to give a white solid (1.0 g). This solid was then dissolved in ca. 50 ml of distilled water and triturated for i h. The white solid was then collected and dried in vacuo at 50° C to give 0.62 g (72 % in two steps) of TFA salt of compound **(B)** as a white solid.
TLC: Rf= 0.19 (10 % CH3OH/CHCL3)
IR (cm⁻¹): 2961, 2935,1741, 1674, 1621, 1503, 1442, 1393, 1281, 1258, 1202, 1144, 1127, 1066, 970.
UV (CH3OH): 230 nm (e= 24,055), 247 nm (e= 24,515).
¹HNMR(300 MHz,CD4OD) d: 7.30 (m, 5H), 7.17 (d, J= 2.1 Hz, 1H), 7.09 (dd, J= 1.9HZ, J= 8.4 Hz, 1H), 6.90 (d, J= 8.4 Hz, 1H), 6.65 (m, 1H), 6.40 (d, J= 15.8 Hz, 1H), 6.02 (m, 2H), 4.93 (m, 2H), 4.53 (m, 1H), 3.79 (s, 3H), 3.85 (m, 1H), 3.57 (m, 1H), 3.14 (m, 2H), 2.90 (m, 2H), 2.62 (m, 4H), 1.42-1.70 (m, 3H), 1.27 (d, J= 6.7 Hz, 3H), 1.10 (d, J= 6.8 Hz, 3H), 0.77 (d, J= 6.4 Hz, 3H), 0.70 (d, J= 6.4 hz, 3H).
OR; [a]_{D}= (CHCl3n c= )
Mass(FD): 785.4 (M⁺).
Anal: Calcd for C38H48N2O10F3Cl (TFA salt): C, 58.12; H,6.16; N,3.57. Found: C, 57.92; H, 6.11, N, 3.91.

### Example 6

### Synthesis of compound (C)

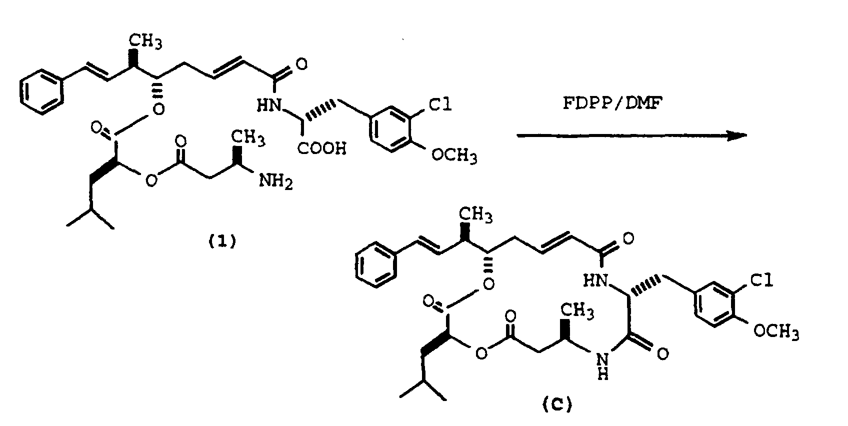

A sample of 0.30 g (0.39 mmol) of **(1)** was dissolved in 50 ml of dry DMF under argon atmosphere and to this was then added 0.19 g (0.51 mmol, 1.3 eq) of pentafluorophenyldiphenylphosphinate (FDPP) in 8 ml of dry DMF. The resulting reaction mixture was then let stirred at room temperature for 5h. DMF was then removed in vacuo and the residue was triturated with ether/hexane to give a crude solid. This solid was then flash chromatographed on SiO2 (5 % CH3OH/CHCl3) to give 0.18 g (72 %) of cyclized compound **(C)** as white solid.
TLC: Rf= 0.21 (10 % CH3OH/CHCL3)
IR (cm⁻¹): 3394, 3290, 2960, 1744, 1728, 1676, 1659, 1539, 1521, 1503, 1442, 1204, 1173, 751.
UV (CH3OH): 247 nm (e= 21,980).
¹HNMR(300 MHz,CDCl3) d: 7.85 (d, J=9.6 Hz, 1H), 7.31 (m, 5H), 7.19 (d, J= 2.0 Hz, 1H), 7.03 (dd, J= 2.0 Hz, J= 8.3 Hz, 1H), 6.83 (d, J= 8.2 Hz, 1H), 6.79 (m, 1H), 6.38 (d, J= 15.9 Hz, 1H), 6.00 (dd, J= 8.8 Hz, J= 15.3 H, 1H), 5.74 (d, J= 14.7hz, 1H), 5.39 (d, J= 8 Hz, 1H), 5.12 (m, 1H), 4.77 (m, 2H), 4.25 (m, 1H), 3.88 (s, 3H), 3.26 (dd, J= 5.8 Hz, J= 13.2 Hz, 1H), 2.94 (dd. J= 5.2 Hz, J= 14.3 Hz, 1H), 2.42 - 2.70 (m, 4H), 1.64 (m, 2H), 1.31 (m, 1H), 1.14 (d, J= 4.4 Hz, 3H), 1.12 (d, J= 4.4 Hz, 3H), 0.74 (d, J= 2.9 Hz, 3H), 0.71 (d, J= 2.9 Hz, 3H).
OR: [a]_{D}= (CHCl3, c= )
Mass(FAB): 639.4 (M⁺).
Anal: Calcd for C35H43N2O7Cl: C, 66.20; H,6.94; N,4.29. Found: C, 66.04; H, 6.82, N, 4.38.

### Example 7

### The synthesis of epoxides (D) and (E)

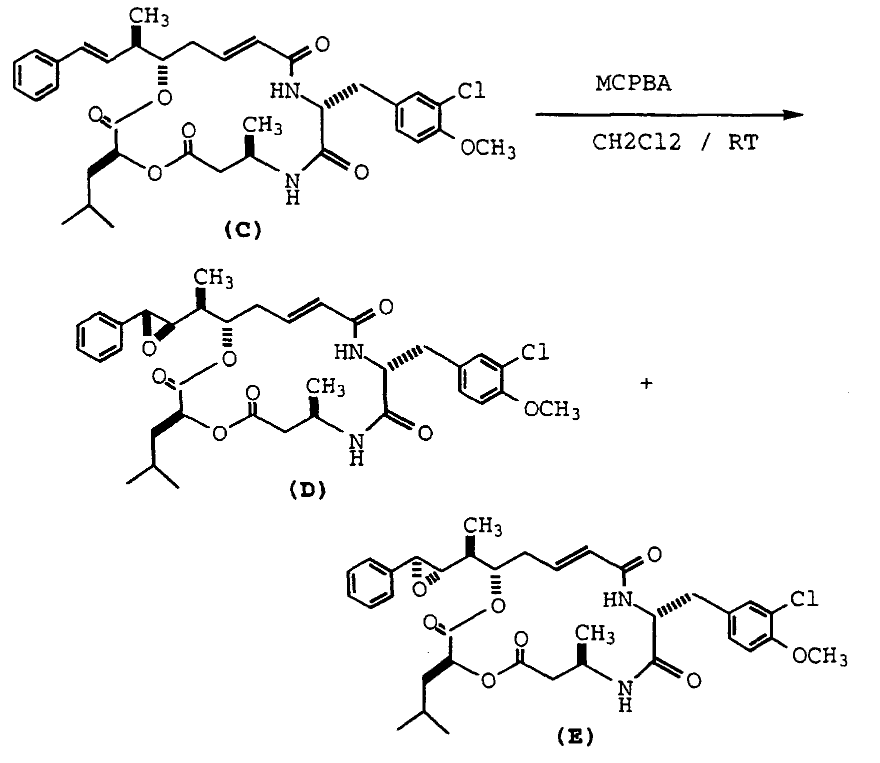

A sample of mg ( mmol) of compound **(C)** was dissolved in ml of dry methylene chloride, to this was then added mg (2.0 eq) of MCPBA at room temperature. This was then let stirred under argon for 18 h. The reaction mixture was checked by HPLC (2X4.6mmX15cm Novapak C-18 column, 75/25 CH3CN:H2O, 1.0 ml/min, monitored at 254 nm) and it was found that ca % of starting material still remain after 18 h. Another mg (1.0 eq) of MCPBA was added and reaction again monitored by HPLC. After stirring overnight (ca 41 h total) reaction, mg of MCPBA was added (total: mg, eq) to the reaction mixture. The reaction was let stirred for another 6 h until HPLC showed only 1.3 % of starting material remained unreacted. HPLC also indicated the two epoxides **(D)** and **(E)** existed in a ratio of ca. :1.0. Of the 5.5ml of the reaction mixture, 1.5 ml of the sample (ca 30 % eq) was removed and used immediately for the chlorohydrin reaction for the next step (see next experimental). Of the remaining 70 % (ca. 4.0 ml) reaction mixture, it was then diluted with methylene chloride (50 ml) and washed with 5%NaHCO3 (20mlX2) to remove the unreacted MCPBA and metachlorobenzoic acid. The organic layer was then dried over Na2SO4 and concentrated in vacuo to give mg of pale yellow solid which was purified and separated on a semiprep reversed phase C-18 HPLC column with the following condition:
column:
   solvent: CH3CN/H2O:
   flow rate: 5.0 ml/min
   UV: 254 nm

This gives the two epoxides **(D)** and **(E)**:

### Example 8

### Synthesis of chlorohydrin compounds (F) and (G)

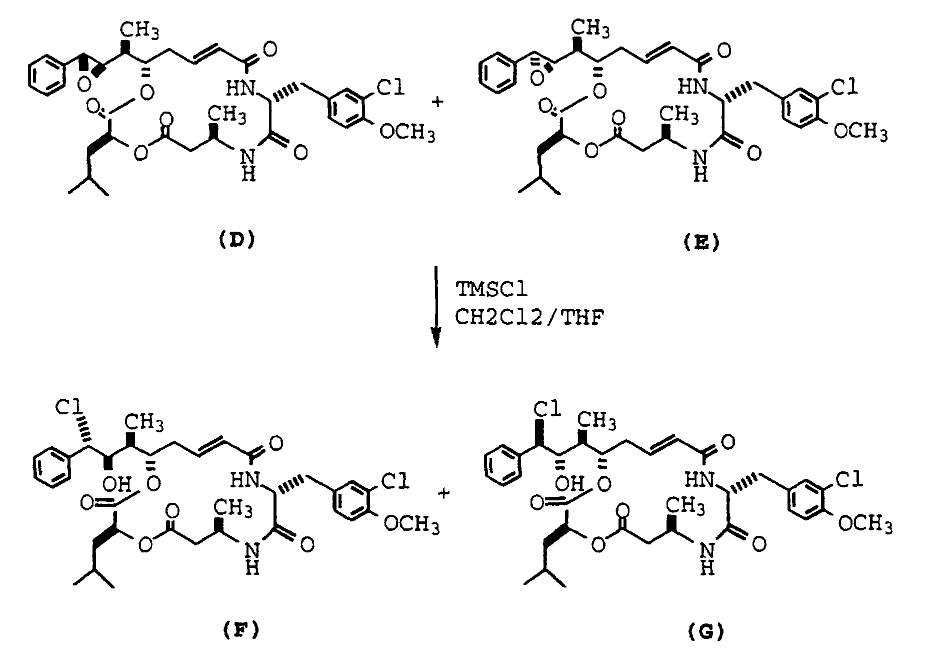

To the ml methylene chloride solution obtained from the previous MCPBA reaction ( % eq, ca mg of epoxides in theory, mmol) was added another 1.0 ml of dry THF. The reaction mixture was then kept under argon atmosphere and cooled to -60 C. To this was added ul ( mg, 5.0 eq) of trimethylchlorosilane. The reaction was then followed by TLC (5 % CH3OH/CHCl3), the reaction was let stirred at -60° C for 3 h before another ul of TMSCl was added. TLC showed the appearance of two more polar spots with ratio of ca 2:1. The reaction was then terminated after stirring at -40 C for another 3 h (TLC still showed some unreacted material). The solvent was then removed in vacuo to give a white solid which was further purified by preparative reversed phase HPLC using condition specified below:
column:
solvent: CH3CN/H2O:
flow rate: 5.0 ml/min
UV: 254 nm

This gives the two chlorohydrins **(F)** and **(G)**:

## Claims

1. A cryptophycin compound of Formula I 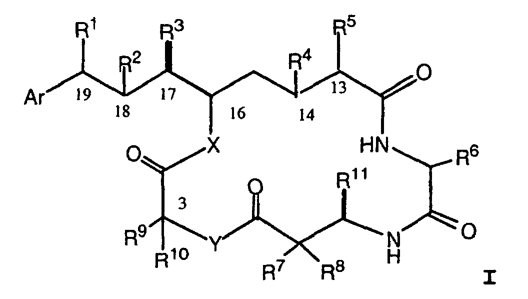 wherein
Ar is selected from the group consisting of phenyl, phenyl with a substituent selected from halogen, simple alkyl and -OR^{12'} wherein R^{12'} is lower alkyl, and an aromatic group selected from furyl, pyrrolyl, thienyl, pyridyl, indolyl and naphthyl which is unsubstituted or substituted by a single group selected from halogen, lower alkyl and -OR^{12'} wherein R^{12'} is a simple alkyl group;
R¹ is selected from the group consisting of halogen, SH, amino, monoalkylamino, dialkylamino, trialkylammonium, alkylthio, dialkylsulfonium, sulfate, or phosphate;
R² is OH or SH; or
R¹ and R² may be taken together with C₁₈ and C₁₉ to form an epoxide ring, and aziridine ring, an episulfide ring, a sulfate ring, or monoalkylphosphate ring; or
R¹ and R² may be taken together to form a second bond between C₁₈ and C₁₉;
R³ is a lower alkyl group;
R⁴ is H or H₂;
R⁵ is H or H₂;
R⁴ and R⁵ may be taken together to form a second bond between C₁₃ and C₁₄;
R⁶ is selected from the group consisting of benzyl, hydroxybenzyl, alkoxybenzyl, halohydroxybenzyl, dihalohydroxybenzyl, haloalkoxybenzyl, and dihaloalkoxybenzyl group;
R⁷ is H or a lower alkyl group;
R⁸ is H, OH or a lower alkyl group;
R⁷ and R⁸ may be taken together to form a spiro group;
R⁹ is H or a lower alkyl group;
R¹⁰ is H or a lower alkyl group;
R¹¹ is selected from the group consisting of simple alkyl
OH, phenyl, phenyl substituted by from one to three substituents independently selected from simple alkyl, OR^{12'} wherein R^{12'} is a simple alkyl group, Cl, Br, F and I, benzyl, and benzyl substituted by from one to three substituents independently selected from simple alkyl, Cl, Br, F and I;
X is O, NH or alkylamino;
Y is O, NH or alkylamino;
simple alkyl means C₁ - C₇ alkyl wherein the alkyl maybe saturated, unsaturated, branched or straight chain; lower alkyl means an alkyl group of one to five carbons; or a pharmaceutically acceptable salt or solvate thereof.

2. A compound of Claim 1 wherein R¹¹ is selected from the group consisting of simple alkyl, phenyl, phenyl substituted by from one to three substituents independently selected from simple alkyl, OR^{12'} wherein R^{12'} is a simple alkyl group, Cl, Br, F and I, benzyl and benzyl substituted by from one to three substituents independently selected from simple alkyl, Cl, Br, F and I;

3. A compound of Claim 1 or 2 wherein Ar is phenyl or substituted phenyl wherein in the phenyl substituents are selected from simple alkyl, and halogen.

4. A compound of any one of Claims 1 to 3 wherein R⁷ and R⁸ are each hydrogen.

5. A compound of any one of Claims 1 to 4 wherein R¹ and R² form an epoxide ring.

6. A compound of any one of Claims 1 to 5 wherein R¹¹ is selected from the group consisting of saturated simple alkyl.

7. A compound of any one of Claims 1 to 5 wherein R¹¹ is selected from the group consisting of unsaturated simple alkyl.

8. A compound of any one of Claims 1 to 5 wherein R¹¹ is selected from the group consisting of benzyl and benzyl substituted by from one to three substituents independently selected from simple alkyl, Cl, Br, F and I.

9. A compound of any one of Claims 1 to 8 wherein Ar is phenyl.

10. Use of a compound of any one of Claims 1 to 9 for the manufacture of a medicament for disrupting a microtubulin system.

11. Use of a compound of any one of Claims 1 to 9 for the manufacture of a medicament for inhibiting the proliferation of a hyperproliferative mammalian cell.

12. A forumulation comprising a compound of any one of Claims 1 to 9 together with one or more pharmaceutically acceptable excipients, diluents, or carriers therefor.

13. A compound of any one of Claims 1 to 9 for use in the treatment of the human or animal body.

14. A compound of Claim 1 wherein the compound is selected from the group consisting of 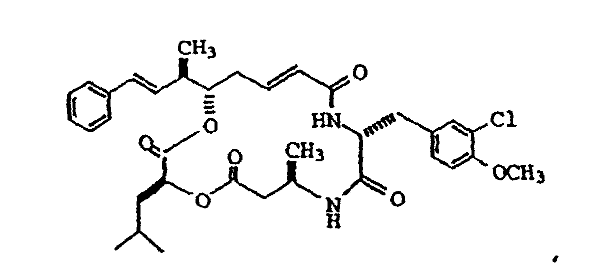 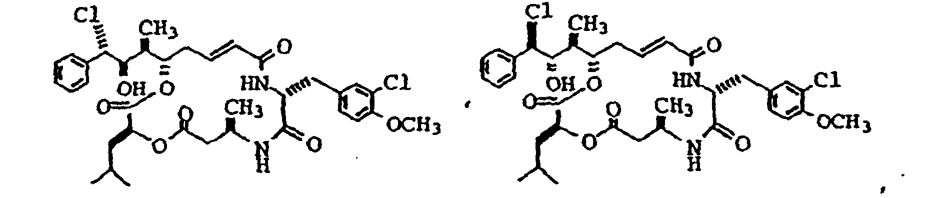 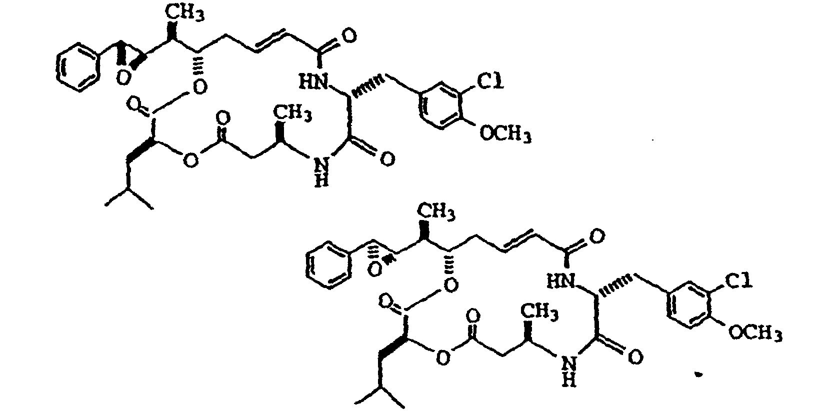 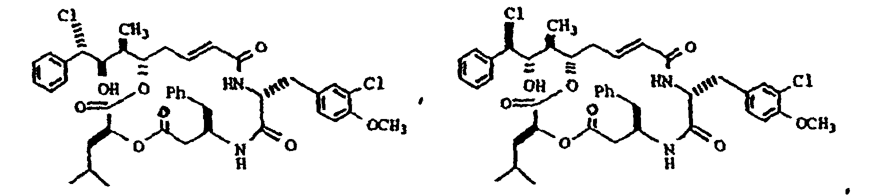 and

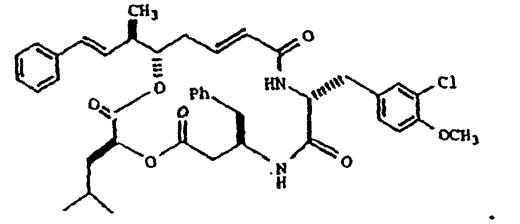

## Patentansprüche

1. Cryptophycinverbindung der Formel I 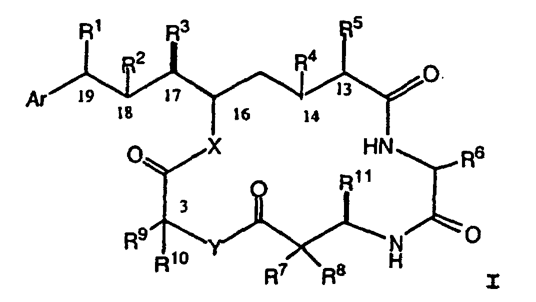 worin
Ar aus der Gruppe ausgewählt ist, die besteht aus Phenyl, Phenyl mit einem Substituenten, ausgewählt aus Halogen, einfachem Alkyl und -OR^{12'}, worin R^{12'} für Niederalkyl steht, und einer aromatischen Gruppe, ausgewählt aus Furyl, Pyrrolyl, Thienyl, Pyridyl, Indolyl und Naphthyl, das unsubstituiert oder substituiert ist durch eine einzelne Gruppe, ausgewählt aus Halogen, Niederalkyl und -OR^{12'}, worin R^{12'} für eine einfache Alkylgruppe steht,
R¹ aus der Gruppe ausgewählt ist, die besteht aus Halogen, SH, Amino, Monoalkylamino, Dialkylamino, Trialkylammonium, Alkylthio, Dialkylsulfonium, Sulfat oder Phosphat,
R² für OH oder SH steht, oder
R¹ und R² mit C₁₈ und C₁₉ unter Bildung eines Epoxidrings, eines Aziridinrings, eines Episulfidrings, eines Sulfatrings oder eines Monoalkylphosphatrings zusammengenommen werden können, oder
R¹ und R² unter Bildung einer zweiten Bindung zwischen C₁₈ und C₁₉ zusammengenommen werden können,
R³ für eine Niederalkylgruppe steht,
R⁴ für H oder H₂ steht,
R⁵ für H oder H₂ steht,
R⁴ und R⁵ unter Bildung einer zweiten Bindung zwischen C₁₃ und C₁₄ zusammengenommen werden können.
R⁶ aus der Gruppe ausgewählt ist, die besteht aus einer Benzyl-, Hydroxybenzyl-, Alkoxybenzyl-, Halogenhydroxybenzyl-, Dihalogenhydroxybenzyl-, Halogenalkoxybenzyl- und Dihalogenalkoxybenzlygruppe,
R⁷ für H oder eine Niederalkylgruppe steht,
R⁸ für H, OH oder eine Niederalkylgruppe steht,
R⁷ und R⁸ unter Bildung einer Spirogruppe zusammengenommen werden können,
R⁹ für H oder eine Niederalkylgruppe steht,
R¹⁰ für H oder eine Niederalkylgruppe steht,
R¹¹ aus der Gruppe ausgewählt ist, die besteht aus einfachem Alkyl, OH, Phenyl, Phenyl, das mit ein bis drei Substituenten substituiert ist, unabhängig ausgewählt aus einfachem Alkyl, OR^{12'}, worin R^{12'} für eine einfache Alkylgruppe, Cl, Br, F und I, Benzyl und Benzyl steht, das mit ein bis drei Substituenten substituiert ist, unabhängig ausgewählt aus einfachem Alkyl, Cl, Br, F und I,
X für O, NH oder Alkylamino steht,
Y für O, NH oder Alkylamino steht
einfaches Alkyl für C₁-C₇ Alkyl steht, worin Alkyl gesättigt, ungesättigt, verzweigt- oder geradkettig sein kann,
Niederalkyl für eine Alkylgruppe mit ein bis fünf Kohlenstoffatomen steht,
oder ein pharmazeutisch annehmbares Salz oder Solvat hiervon.

2. Verbindung nach Anspruch 1, worin R¹¹ aus der Gruppe ausgewählt ist, die besteht aus einfachem Alkyl, Phenyl und Phenyl, das mit ein bis drei Substituenten substituiert ist, unabhängig ausgewählt aus einfachem Alkyl, -OR^{12'}, worin R^{12'} für eine einfache Alkylgruppe, Cl, Br, F und I, Benzyl und Benzyl steht, das substituiert ist mit 1 bis 3 Substituenten, unabhängig ausgewählt aus einfachem Alkyl, Cl, Br, F und I.

3. Verbindung nach Anspruch 1 oder 2, worin Ar für Phenyl oder substituiertes Phenyl steht, worin die Phenylsubstituenten aus einfachem Alkyl und Halogen ausgewählt sind.

4. Verbindung nach einem der Ansprüche 1 bis 3, worin R⁷ und R⁸ jeweils für Wasserstoff stehen.

5. Verbindung nach einem der Ansprüche 1 bis 4, worin R¹ und R² einen Epoxidring bilden.

6. Verbindung nach einem der Ansprüche 1 bis 5, worin R¹¹ aus der Gruppe ausgewählt ist, die besteht aus gesättigtem, einfachem Alkyl.

7. Verbindung nach einem der Ansprüche 1 bis 5, worin R¹¹ aus der Gruppe ausgewählt ist, die besteht aus ungesättigtem, einfachem Alkyl.

8. Verbindung nach einem der Ansprüche 1 bis 5, worin R¹¹ aus der Gruppe ausgewählt ist, die besteht aus Benzyl und Benzyl, das mit ein bis drei Substituenten substituiert ist, unabhängig ausgewählt aus einfachem Alkyl, Cl, Br, F und I.

9. Verbindung nach einem der Ansprüche 1 bis 8, worin Ar für Phenyl steht.

10. Verwendung einer Verbindung nach einem der Ansprüche 1 bis 9 zur Herstellung eines Arznemittels zur Zerstörung eines Mikrotubulinsystems.

11. Verwendung einer Verbindung nach einem der Ansprüche 1 bis 9 zur Herstellung eines Arzneimittels zur Hemmung der Proliferation einer hyperproliferativen Säugerzelle.

12. Formulierung, die eine Verbindung nach einem der Ansprüche 1 bis 9 zusammen mit einem oder mehreren pharmazeutisch annehmbaren Hilfsstoffen, Verdünnungsmitteln oder Trägern hierfür enthält.

13. Verbindung nach einem der Ansprüche 1 bis 9 zur Verwendung bei der Behandlung des menschlichen oder tierischen Körpers.

14. Verbindung nach Anspruch 1, worin die Verbindung aus der Gruppe ausgewählt ist, die besteht aus 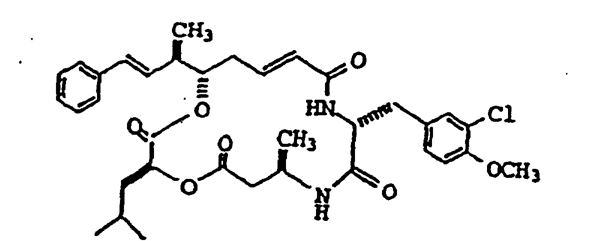 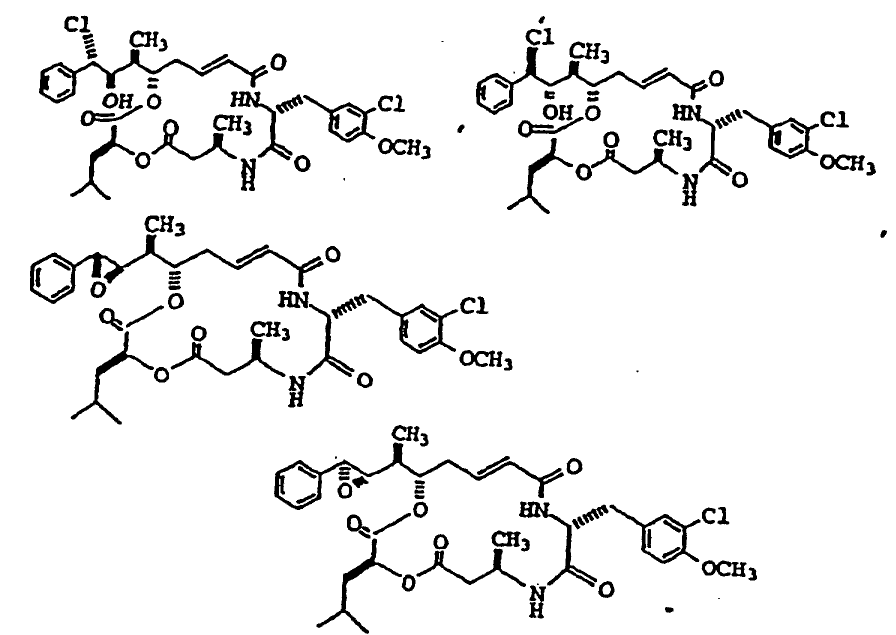 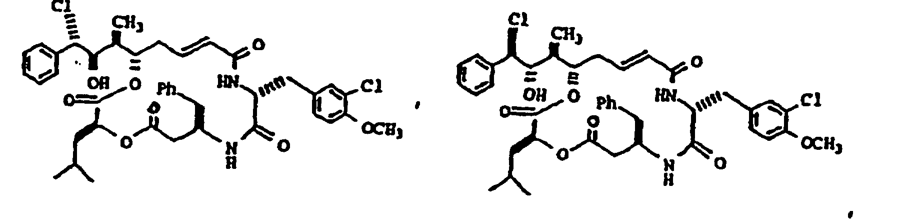 und

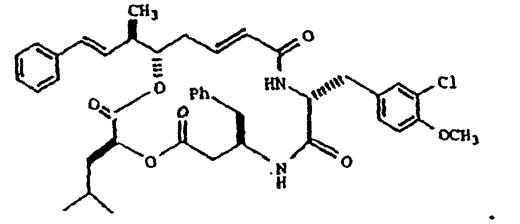

## Revendications

1. Composé de cryptophycine répondant à la formule I 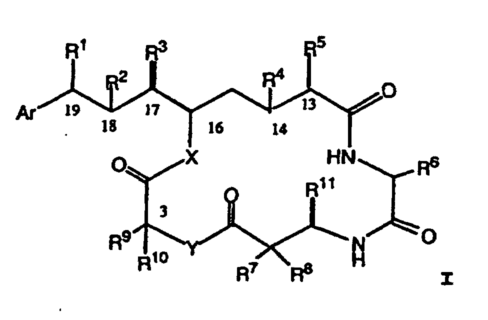 dans laquelle
Ar est choisi parmi le groupe constitué par un groupe phényle, par un groupe phényle portant un substituant choisi parmi le groupe comprenant un atome d'halogène, un groupe alkyle simple et un groupe -OR^{12'} dans lequel R^{12'} représente un groupe alkyle inférieur, et par un groupe aromatique choisi parmi le groupe comprenant un groupe furyle, un groupe pyrrolyle, un groupe thiényle, un groupe pyridyle, un groupe indolyle et un groupe naphtyle qui est non substitué ou substitué par un groupe unique choisi parmi le groupe comprenant un atome d'halogène, un groupe alkyle inférieur et un groupe - OR^{12'} dans lequel R^{12'} représente un groupe alkyle simple ;
R¹ est choisi parmi le groupe constitué par un atome d'halogène, un groupe SH, un groupe amino, un groupe monoalkylamino, un groupe dialkylamino, un groupe trialkylammonium, un groupe alkylthio, un groupe dialkylsulfonium, un groupe sulfate et un groupe phosphate ;
R² représente un groupe OH ou un groupe SH ; ou bien
R¹ et R² peuvent être pris ensemble avec l'atome de carbone en position 18 et l'atome de carbone en position 19 pour former un noyau époxyde, un noyau aziridine, un noyau épisulfure, un noyau sulfate ou un noyau monoalkylphosphate ; ou bien
R¹ et R² peuvent être pris ensemble pour former une deuxième liaison entre l'atome de carbone en position 18 et l'atome de carbone en position 19 ;
R³ représente un groupe alkyl inférieur ;
R⁴ représente un atome d'hydrogène ou un groupe H₂ ;
R⁵ représente un atome d'hydrogène ou un groupe H₂;
R⁴ et R⁵ peuvent être pris ensemble pour former une deuxième liaison entre l'atome de carbone en position 13 et l'atome de carbone en position 14 ;
R⁶ est choisi parmi le groupe constitué par un groupe benzyle, un groupe hydroxybenzyle, un groupe alcoxybenzyle, un groupe halogénohydroxybenzyle, un groupe dihalogénohydroxybenzyle, un groupe halogénoalcoxybenzyle et un groupe dihalogénoalcoxybenzyle ;
R⁷ représente un atome d'hydrogène ou un groupe alkyle inférieur ;
R⁸ représente un atome d'hydrogène, un groupe OH ou un groupe alkyle inférieur ;
R⁷ et R⁸ peuvent être pris ensemble pour former un groupe spiro ;
R⁹ représente un atome d'hydrogène ou un groupe alkyle inférieur ;
R¹⁰ représente un atome d'hydrogène ou un groupe alkyle inférieur ;
R¹¹ est choisi parmi le groupe constitué par un groupe alkyle simple, un groupe OH, un groupe phényle, un groupe phényle portant de un à trois substituants choisis de manière indépendante parmi le groupe comprenant un groupe alkyle simple, un groupe -OR^{12'} dans lequel R^{12'} représente un groupe alkyle simple, un atome de chlore, un atome de brome, un atome de fluor et un atome d'iode, un groupe benzyle et un groupe benzyle portant de un à trois substituants choisis de manière indépendante parmi le groupe comprenant un groupe alkyle simple, un atome de chlore, un atome de brome, un atome de fluor et un atome d'iode ;
X représente un atome d'oxygène, un groupe NH ou un groupe alkylamino ;
Y représente un atome d'oxygène, un groupe NH ou un groupe alkylamino ;
l'expression "groupe alkyle simple" désigne un groupe alkyle en C₁-C₇ dans lequel le groupe alkyle peut être saturé, insaturé, à chaîne droite ou ramifiée;
l'expréssion "groupe alkyle inférieur" désigne un groupe alkyle contenant de 1 à 5 atomes de carbone ;
ou un de ses sels ou de ses solvates pharmaceutiquement acceptables.

2. Composé selon la revendication 1, dans lequel R¹¹ est choisi parmi le groupe constitué par un groupe alkyle simple, un groupe phényle, un groupe phényle portant de un à trois substituants choisis de manière indépendante parmi le groupe comprenant un groupe alkyle simple, un groupe -OR^{12'} dans lequel R^{12'} représente un groupe alkyle simple, un atome de chlore, un atome de brome, un atome de fluor et un atome d'iode, un groupe benzyle et un groupe benzyle portant de un à trois substituants choisis de manière indépendante parmi le groupe comprenant un groupe alkyle simple, un atome de chlore, un atome de brome, un atome de fluor et un atome d'iode.

3. Composé selon la revendication 1 ou 2, dans lequel Ar représente un groupe phényle ou un groupe phényle substitué, dans lequel les substituants du groupe phényle sont choisis parmi le groupe comprenant un groupe alkyle simple et un atome d'halogène.

4. Composé selon l'une quelconque des revendications 1 à 3, dans lequel R⁷ et R⁸ représentent chacun un atome d'hydrogène.

5. Composé selon l'une quelconque des revendications 1 à 4, dans lequel R¹ et R² forment un noyau époxyde.

6. Composé selon l'une quelconque des revendications 1 à 5, dans lequel R¹¹ est choisi parmi le groupe constitué par un groupe alkyle simple saturé.

7. Composé selon l'une quelconque des revendications 1 à 5, dans lequel R¹¹ est choisi parmi le groupe constitué par un groupe alkyle simple insaturé.

8. Composé selon l'une quelconque des revendications 1 à 5, dans lequel R¹¹ est choisi parmi le groupe constitué par un groupe benzyle et un groupe benzyle portant de un à trois substituants choisis de manière indépendante parmi le groupe comprenant un groupe alkyle simple, un atome de chlore, un atome de brome, un atome de fluor et un atome d'iode.

9. Composé selon l'une quelconque des revendications 1 à 8, dans lequel Ar représente un groupe phényle.

10. Utilisation d'un composé selon l'une quelconque des revendications 1 à 9 pour la préparation d'un médicament destiné à interrompre un système de microtubuline.

11. Utilisation d'un composé selon l'une quelconque des revendications 1 à 9 pour la préparation d'un médicament destiné à inhiber la prolifération d'une cellule mammalienne hyperproliférante.

12. Formulation comprenant un composé selon l'une quelconque des revendications 1 à 9, en association avec un ou plusieurs excipients, diluants ou supports pharmaceutiquement acceptables pour ledit composé.

13. Composé selon l'une quelconque des revendications 1 à 9, à utiliser dans le traitement du corps humain ou animal.

14. Composé selon la revendication 1, dans lequel le composé est choisi parmi le groupe constitué par 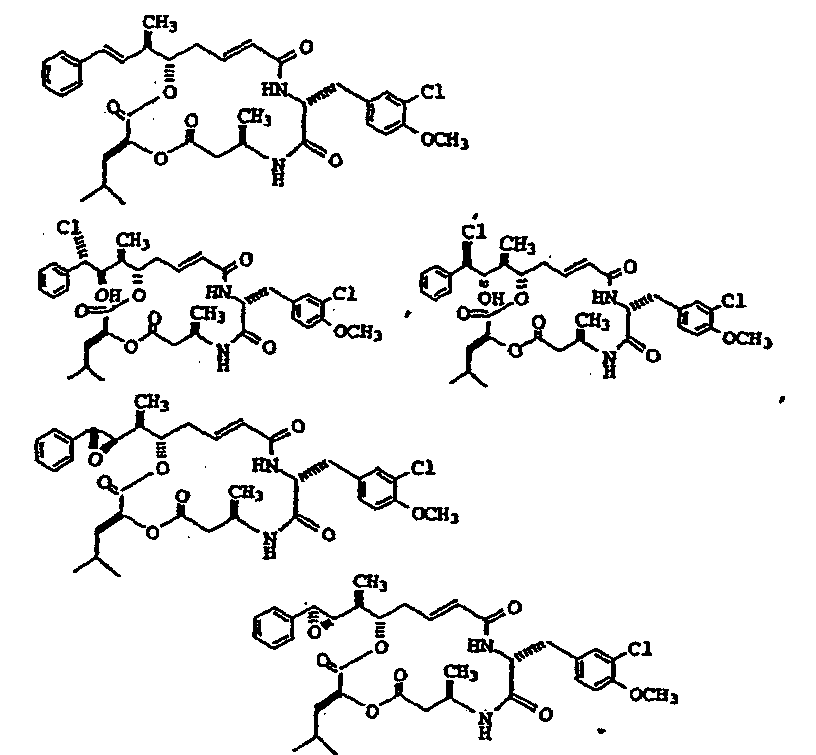 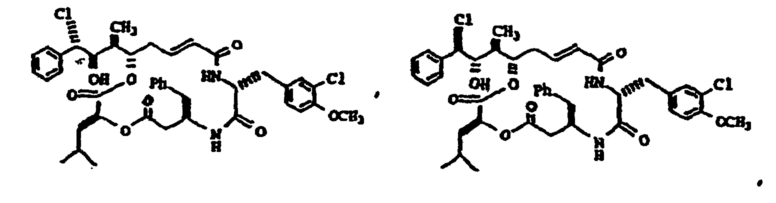 et
